# FASCICULE DE BREVET EUROPEEN

(11) **EP 1 100 801 B1**
(45) Date de publication et mention de la délivrance du brevet: **03.03.2010**
(21) Numéro de dépôt: 99929394.7
(22) Date de dépôt: 05.07.1999
(51) Int. Cl.: C07D 487/04, A61K 31/495, C07D 241/00, C07D 235/00

(54) **UTILISATION DE DERIVES DE LA CYSTEINE POUR PREPARER UN MEDICAMENT DESTINE A TRAITER LES PATHOLOGIES QUI RESULTENT DE LA FORMATION DE LA PROTEINE G HET**
ANWENDUNG VON CYSTEIN-DERIVATEN ZUR HERSTELLUNG EINES ARZNEIMITTELS BESTIMMT ZUR BEHANDLUNG VON PATHOLOGIEN HERVORGEHEND AUS ENTSTEHUNG DER PROTEIN G HET
USE OF CYSTEINE DERIVATIVES FOR PREPARING A MEDICINE FOR TREATING PATHOLOGIES RESULTING FROM THE FORMATION OF HETEROTRIMERIC G PROTEIN

(30) Priorité: 08.07.1998 FR 9808731
(43) Date de publication de la demande: 23.05.2001
(73) Titulaire: IPSEN PHARMA, 92100 Boulogne-Billancourt (FR)
(72) Inventeur: PREVOST, Grégoire, F-92160 Antony (FR); LONCHAMPT, Marie-Odile, F-94550 Chevilly-Larue (FR); GORDON, Thomas, Medway, MA 02053 (US)
(74) Mandataire: Bourgouin, André
(86) Numéro de dépôt international: PCT/FR1999/001609
(87) Numéro de publication internationale: WO 2000/002881

(56) Documents cités:
- WO-A-96/21456
- WO-A-97/30053
- WO-A-97/38697
- FR-A- 2 736 638
- US-A- 5 705 686

## Description

La présente invention concerne notamment l'utilisation de dérivés de la cystéine pour préparer un médicament destiné à traiter des pathologies qui résultent de la formation de la protéine G hétérotrimérique. Ces maladies comprennent en particulier des maladies liées aux fonctions biologiques ou désordres suivants : odorat, goût, perception de la lumière, neurotransmission, neurodégénérescence, fonctionnement des glandes endocrines et exocrines, régulation autocrine et paracrine, tension artérielle, embryogénèse, prolifération cellulaire bénigne, oncogénèse, infection virale, fonctions immunologiques, diabète, obésité, et maladies prolifératives bénignes et malignes.

Les protéines G sont en fait l'association structurale de trois sous-unités distinctes appelées α, β et γ, mais fonctionnent comme des entités dissociables constituées par des sous-unités α d'un côté et des dimères β / γ de l'autre.

Les protéines G participent à la transmission de signaux de l'extérieur de la cellule grâce à son interaction avec les récepteurs à sept domaines transmembranaires vers l'intérieur par l'intermédiaire de différents effecteurs incluant l'adénylate cyclase, la phospholipase C ou encore les canaux ioniques. L'enzyme adénylate cyclase génère de l'AMP cyclique (AMPc) (cf. Gilman, A.G. Biosci.Rep. 15, 65-97 (1995)). Ainsi, on sait que pour activer l'adénylate cyclase, il est nécessaire que les protéines G soient transitoirement dans une forme hétérotrimérique, forme dans laquelle le monomère constitué par une sous-unité α est associée au dimère constitué par les sous-unités β et γ. C'est uniquement dans cette situation que le signal de l'extérieur de la cellule peut activer la sous-unité α d'une protéine G, laquelle pourra après dissociation moduler l'adénylate cyclase et moduler la production d'AMPc.

On sait aussi que les dimères β / γ peuvent activer directement des effecteurs conduisant à l'activation de kinases régulées par des signaux extracellulaires (ERKs) ou des MAP kinases. Un lien direct entre les sous-unités β / γ et les kinases src ou *src like* a été démontré (cf. Gutkind, J.S. J.Biol.Chem. 273, 1839-1842 (1998)).

Par ailleurs, des toxines bactériennes comme*Vibrio cholera* et *Bortella pertussis*, des peptides comme le mastoparan et la suramine ont été présentés comme modulant directement l'activité des protéines G (cf. Freissmuth, M., Boehm, S., Beindl, W., et coll. Mol.Pharmacol. 49, 602-611 (1996); Boehm, S., Huck, S., Motejlek, A., et coll. Journal of Neurochemistry 66, 1019-1026 (1996) ; Cachero, T.G., Rigual, R., Rocher, A. & Gonzalez, C. Eur.J.Neurosci. 8, 2320-2327 (1996) ; Danilenko, M., Worland, P., Carlson, B., Sausville, E.A. & Sharoni, Y. Biochem.Biophys.Res. Commun. 196, 1296-1302 (1993) ; Beindl, W., Mitterauer, T., Hohenegger, M., Ijzerman, A.P., Nanoff, C. & Freissmuth, M. Mol.Pharmacol. 50, 415-423 (1996)).

Par exemple, la toxine cholérique modifie la sous-unité α_{S} de la protéine G par addition d'un ADP-ribose provenant du NAD à un site accepteur spécifique arginine. Ceci bloque complètement l'activité de la GTPase, provoquant une stimulation persistante de son effecteur suivant, l'adénylate cyclase et conduisant à une surproduction d'AMPc.

Les effets néfastes d'un taux anormal d'AMPc sont également connus et ont notamment lieu au niveau des fonctions biologiques ou désordres suivants : odorat, goût, perception de la lumière, neurotransmission, neurodégénérescence, fonctionnement des glandes endocrines et exocrines, régulation autocrine et paracrine, tension artérielle, embryogénèse, prolifération cellulaire bénigne, oncogénèse, infection virale et fonctions immunologiques, diabète et obésité.

Le brevet US 5,705,686 décrit des inhibiteurs de farnésyltransférase p21ras de formule générale C¹R dans laquelle C¹ est 3-mercapto-2-amino-propylamino et R est un groupe biphényle éventuellement substitué par un groupe carboxyle et/ou un groupe méthyle. Ces composés peuvent être utilisés dans le traitement du cancer.

La demande de brevet PCT WO 97/38697 décrit entre autres l'inhibiteur de géranylgéranyltransférases GGTI-286

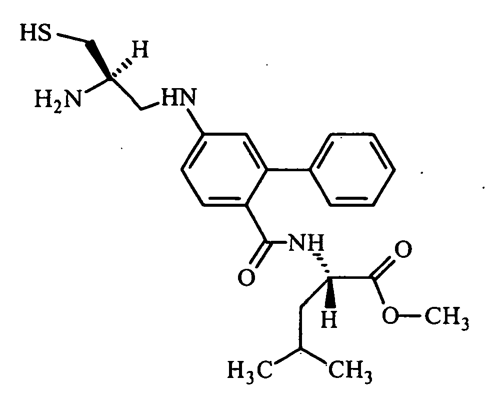

Ce composé est utilisable dans le traitement du cancer grâce à ses propriétés de sensibilisation des cellules aux radiations.

Par ailleurs, la demande de brevet français FR 2 736 638 concerne des inhibiteurs de farnésyltransférase de formule générale 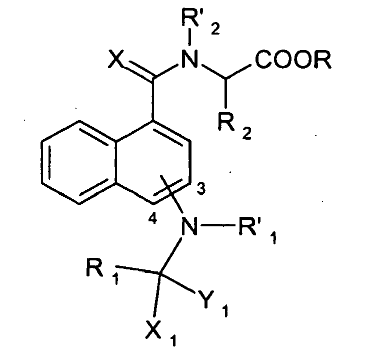 dans laquelle Y représente un radical Y-S-A₁- dans lequel Y est notamment H ou un radical R₄-S- dans lequel R₄ représente notamment un radical de formule générale 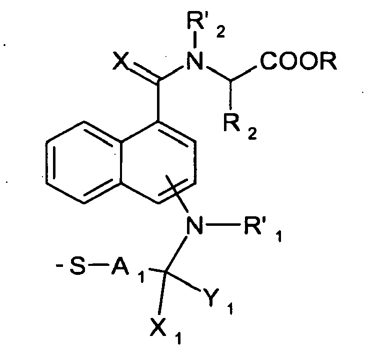 dans laquelle A₁, X, X₁, Y₁, R'₁, R₂, R'₂ et R sont définis comme ci-après,
A₁ représente un radical alcoylène en C₁-C₄ éventuellement substitué en α du groupement >C(X₁)(Y₁) (notamment par un radical amino, alkylamino ou dialkylamino),
X₁ et Y₁ représentent chacun un atome d'hydrogène ou forment ensemble avec l'atome de carbone auquel ils sont liés un groupement >C=O,
R'₁ représente un atome d'hydrogène ou un radical alkyle en C₁-C₆,
X représente un atome d'oxygène ou de soufre,
R₂ représente un radical alkyle, alkényle ou alkynyle en C₁-C₆ éventuellement substitué par un radiocal hydroxy, alkoxy en C₁-C₄, mercapto, alkylthio en C₁-C₄, alkylsulfinyle en C₁-C₄ ou alkylsulfonyle en C₁-C₄, étant entendu que lorsque R_{é} représente un radical alkyle substitué par un radical hydroxy, R₂ peut former avec le radical carboxy en α une lactone,
R'₂ représente un atome d'hydrogène ou un radical alkyle en C₁-C₆,
R représente un atome d'hydrogène ou un radical alcoyle en C₁-C₆ éventuellement substitué par un radical alkoxy en C₁-C₄, alkylthio en C₁-C₄, alkylsulfinyle en C₁-C₄, alkylsulfonyle en C₁-C₄, phényle éventuellement substitué, phénoxy, phénylthio, phénylsulfinyl, phénylslfonyl, alcoylamino en C₁-C₄, dialcoylamino dont chaque partie alcoyle est en C₁-C₄,
étant entendu que le radical 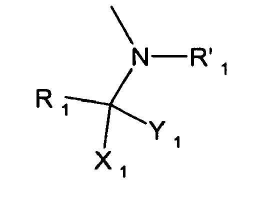 est en position 3 ou 4 du noyau naphtyle.

Ces composés sont décrits en tant qu'agents anti-cancéreux qui bloquent la capacité de la protéine Ras mutée à transformer les cellules normales en cellules cancéreuses.

La demanderesse vient de découvrir que certains dérivés de la cystéine, à savoir les composés de formule générale **(A)** 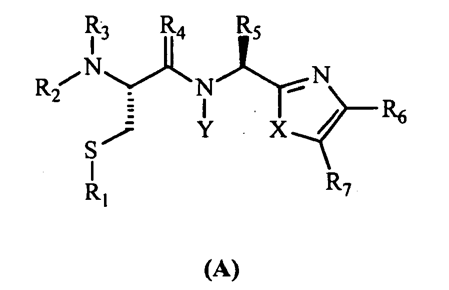 correspondant aux sous-formules **(A1)** ou **(A2) :** 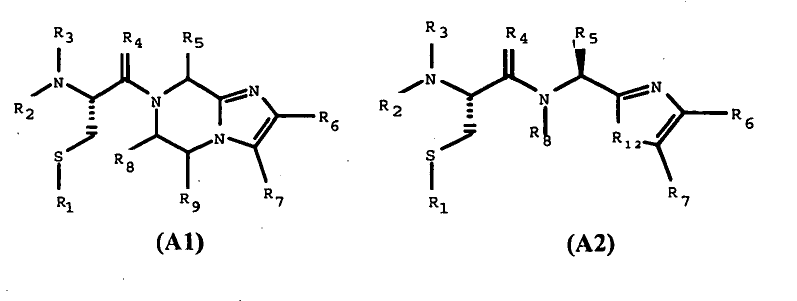 dans lesquelles :
X représente R₁₂ et Y représente R₈, ou X et Y complètent un cycle à 6 chaînons, l'ensemble X-Y représentant le radical -CH(R₈)-CH(R₉)- ;
R₁ représente H, un radical alkyle comptant de 1 à 6 atomes de carbone ou alkylthio comptant de 1 à 6 atomes de carbone ;
R₂ et R₃ représentent indépendamment H ou un radical alkyle comptant de 1 à 6 atomes de carbone ;
R₄ représente H₂ ou O ;
R₅ représente H, ou l'un des radicaux alkyle comptant de 1 à 6 atomes de carbone alkénule comptant jusqu'à 6 atomes de carbone, alkényle comptant jusqu'à 6 atomes de carbone, alkynyle comptant jusqu'à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé d'un radical alkyle comptant de 1 à 6 atomes de carbone, , -OR₁₀, -S(O)ₘR₁₀ (m représentant 0, 1, ou 2), -N(R₁₀)(R₁₁), -N-C(O)-R₁₀, -NH-(SO₂)-R₁₀, - CO₂-R₁₀, C(O)-N(R₁₀)(R₁₁), et -(SO₂)-N(R₁₀)(R₁₁) ;
R₆ et R₇ représentent indépendamment H, un radical -C(O)-NH-CHR₁₃-CO₂R₁₄, ou l'un des radicaux alkylecomptant de 1 à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé des radicaux OH, alkyle ou alkoxy comptant de 1 à 6 atomes de carbone, N(R₁₀)(R₁₁), COOH, CON(R₁₀)(R₁₁), et halo,
   ou R₆ et R₇ forment ensemble un radical aryle ou un hétérocycle ;
R₈ et R₉ représentent indépendamment, H, ou l'un des radicaux alkyle comptant de 1 à 6 atomes de carbone, aryle, arylalkyle dont le radical alkyle compte de 1 à 6 atomes de carbone, hétérocycle ou hétérocycle alkyle comptant de 1 à 6 atomes de carbone, ces radicaux pouvant éventuellement être substitués par des radicaux choisis parmi le groupe composé des radicaux OH, alkyle ou alkoxy comptant de 1 à 6 atomes de carbone, N(R₁₀)(R₁₁), COOH, CON(R₁₀)(R₁₁) et halo,
ou R₈ et R₉ forment ensemble un radical aryle ou un hétérocycle ;
R₁₀ et R₁₁, représentent indépendamment H, un radical aryle ou hétérocycle, ou un radical alkyle comptant de 1 à 6 atomes de carbone, arylalkyle dont le radical alkyle compte de 1 à 6 de carbone ou hétérocycle alkyle dont le radical alkyle compte de 1 à 6 atomes de carbone ;
R₁₂ représente NR₉, S, ou O ;
R₁₃ représente un radical alkyle inférieur éventuellement substitué par un radical choisi parmi les radicaux alkylecomptant de 1 à 6 atomes de carbone, -OR₁₀, -S(O)ₘR₁₀ (m représentant 0, 1, ou 2) et -N(R₁₀)(R₁₁) ;
R₁₄ représente H ou un radical alkyle comportant de 1 à 6 atomes de carbone ;
étant entendu que les composés de formule générale (A) peuvent également se présenter sous forme de dimères, lorsque deux radicaux R₁ représentant chacun un atome d'hydrogènesont éliminés par oxydation ;
ou d' un sel pharmaceutiquement acceptable d'un composé de formule générale **(A) ;**
utilisé pour préparer un médicament destiné à traiter des pathologies qui résultent de la formation de la protéine G hétérotrimérique à l'exclusion toutefois du cancer et des maladies prolifératives.

Par radical alkyle inférieur, on entend un radical alkyle linéaire ou ramifié comptant de 1 à 6 atomes de carbone, et notamment les radicaux méthyle, éthyle, propyle, isopropyle, butyle, isobutyle, sec-butyle et tert-butyle, pentyle, néopentyle, isopentyle, hexyle, isohexyle. Par radical hétérocycle, on entend un radical constitué de un ou plusieurs cycles et incluant au moins un hétéroatome. Par radical arylalkyle, hétérocycle alkyle, alkylthio ou alkoxy inférieur, on entend les radicaux dont le radical alkyle a la signification indiquée précédemment.

En particulier, les composés suivants peuvent être utilisés pour préparer des médicaments destinés à traiter des pathologies qui résultent de la formation de la protéine G hétérotrimérique :
- la 7-(2-amino-1-oxo-3-thiopropyl)-8-(cyclohexylméthyl)-2-(2-méthylphényl)-5,6,7,8-tétrahydroimidazo[1,2a]pyrazine ;
- la 7-(2-amino-1-oxo-3-thiopropyl)-8-butyl-2-(2-méthoxyphényl)-5,6,7,8-tétrahydroimidazo[1,2a]pyrazine ;
- la 7-(2-amino-1-oxo-3-thiopropyl)-8-(1-méthylpropyl)-2-(2-méthoxyphényl)-5,6,7,8-tétrahydroimidazo[1,2a]pyrazine ;
- la 1-[2(R)-amino-3-mercaptopropyl]-2(S)-n-butyl-4-(1-naphtoyl)pipérazine ;
- le disulfure de bis-1,1'-[7-(2-amino-1-oxo-3-thiopropyl)-2-(méthoxyphényl)-8-(1-méthylpropyl)-5,6,7,8-tétrahydroimidazo[1,2a]pyrazine] ;
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine ;
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) ;
- le composé de formule : 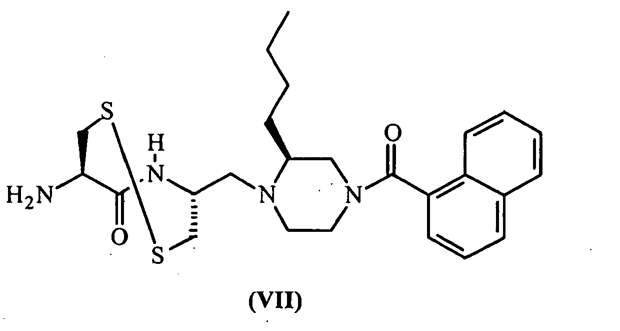
- le composé de formule : 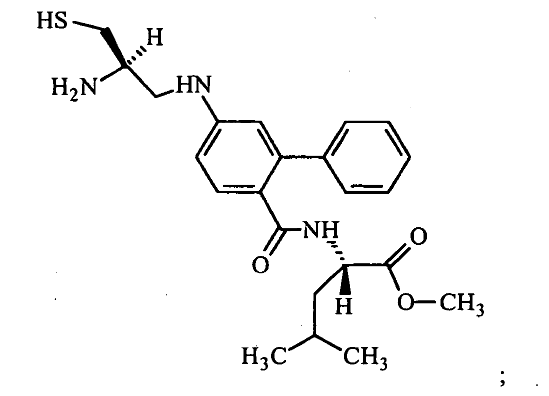
- la 7-(2-amino-1-oxo-3-thiopropyl)-8-(cyclohexylméthyl)-2-phényl-5,6,7,8-tétrahydroimidazo[1,2a]pyrazine ;
- la 7-(2-amino-1-oxo-3-thiopropyl)-2-(2-méthoxyphényl)-8-(phénylméthoxy)méthyl-5,6,7,8-tétrahydroimidazo[1,2a]pyrazine ;
- la 7-(2-amino-1-oxo-3-thiopropyl)-2-(2-méthoxyphényl)-8-(1-phénylméthoxy)éthyl-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydro-imidazo[1,2a]pyrazine, ou sa forme dimérique ;
- et la 7-(2-amino-1-oxo-3-thiopropyl)-2-(2-méthoxyphényl)-8-(phénylsulfonyléthyl)-5,6,7,8-tétrahydro-imidazo[1,2a]pyrazine ;
ou encore un sel pharmaceutiquement acceptable d'un de ces composés.

On utilisera de préférence l'un des composés suivants pour l'invention :
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine **(I) ;**
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) **(II) ;**
- la 7-(2-amino-1-oxo-3-thiopropyl)-8-(cyclohexylméthyl)-2-(2-méthylphényl)-5,6,7,8-tétrahydroimidazo[1,2a]pyrazine **(III) ;**
- le composé de formule : 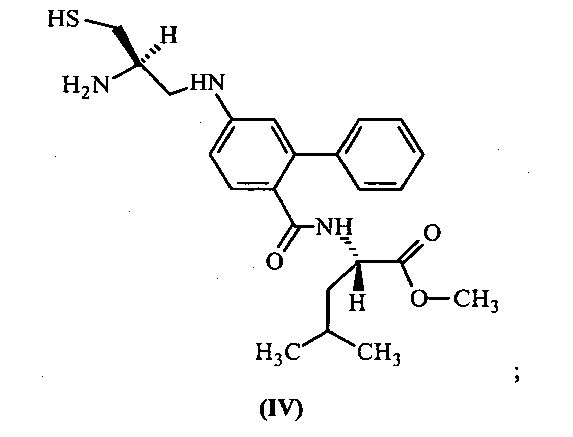
- la 7-(2-amino-1-oxo-3-thiopropyl)-8-butyl-2-(2-méthoxyphényl)-5,6,7,8-tétrahydroimidazo[1,2a]pyrazine **(V) ;**
- le disulfure de bis-1,1'-[7-(2-amino-1-oxo-3-thiopropyl)-2-(méthoxyphényl)-8-(1-méthylpropyl)-5,6,7,8-tétrahydroimidazo[1,2a]pyrazine] **(VI) ;**
- le composé de formule : 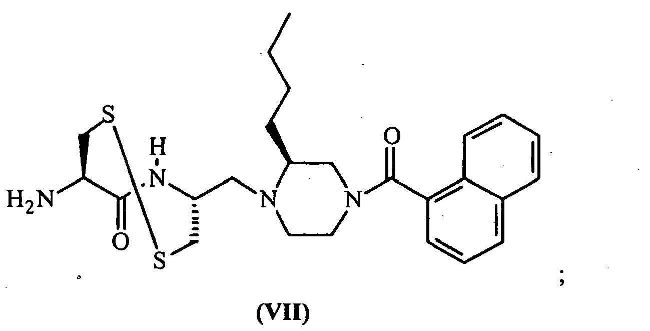
- la 7-(2-amino-1-oxo-3-thiopropyl)-8-(cyclohexylméthyl)-2-phényl-5,6,7,8-tétrahydroimidazo[1,2a]pyrazine ;
- la 7-(2-amino-1-oxo-3-thiopropyl)-8-(1-méthylpropyl)-2-(2-méthoxyphényl)-5,6,7,8-tétrahydroimidazo[1,2a]pyrazine ;
- la 1-[2(R)-amino-3-mercaptopropyl]-2(S)-n-butyl-4-(1-naphtoyl)pipérazine.
- ou un sel pharmaceutiquement acceptable d'un de ces derniers.

Plus préférentiellement, l'un des composés suivants sera utilisé pour l'invention :
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine **(I) ;**
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) **(II) ;**
- la 7-(2-amino-1-oxo-3-thiopropyl)-8-(cyclohexylméthyl)-2-(2-méthylphényl)-5,6,7,8-tétrahydroimidazo[1,2a]pyrazine **(III) ;**
- la 7-(2-amino-1-oxo-3-thiopropyl)-8-butyl-2-(2-méthoxyphényl)-5,6,7,8-tétrahydroimidazo[1,2a]pyrazine **(V) ;**
- le composé de formule : 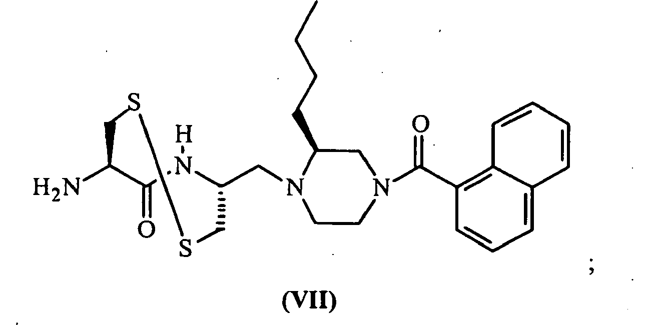
- ou un sel pharmaceutiquement acceptable d'un de ces derniers.

Enfin, les composés suivants seront plus particulièrement préférés :
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine **(I) ;**
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) **(II) ;**
- ou un sel pharmaceutiquement acceptable d'un de ces derniers.

L'invention concerne donc tout d'abord l'utilisation des composés de formule générale **(A)** tels que décrits précédemment pour préparer un médicament destiné à traiter des pathologies qui résultent de la formation de la protéine G hétérotrimérique. En particulier, elle concerne l'utilisation desdits inhibiteurs pour préparer des médicaments destinés à traiter des maladies liées aux fonctions biologiques ou désordres suivants : odorat, goût, perception de la lumière, neurotransmission, neurodégénérescence, fonctionnement des glandes endocrines et exocrines, régulation autocrine et paracrine, tension artérielle, embryogénèse, infection virale, fonctions immunologiques, diabète et obésité.

De façon plus particulière, l'invention concerne l'utilisation des composés de formule générale **(A)** pour préparer un médicament destiné à traiter le choléra, le Syndrome Immuno-Déficitaire Acquis (SIDA), la diarrhée du voyageur et la puberté précoce familiale masculine.

L'invention a aussi pour objet de nouveaux produits de formule générale **(A)** numérotés de 1 à 7 et décrits ci-après dans les exemples, à savoir :
- la 7-(2-amino-1-oxo-3-thiopropyl)-8-(cyclohexylméthyl)-2-(2-méthylphényl)-5,6,7,8-tétrahydroimidazo[1,2a]pyrazine ;
- la 7-(2-amino-1-oxo-3-thiopropyl)-8-(cyclohexylméthyl)-2-phényl-5,6,7,8-tétrahydroimidazo[1,2a]pyrazine ;
- la 7-(2-amino-1-oxo-3-thiopropyl)-2-(2-méthoxyphényl)-8-(phénylméthoxy)méthyl-5,6,7,8-tétrahydroimidazo[1,2a]pyrazine ;
- la 7-(2-amino-1-oxo-3-thiopropyl)-2-(2-méthoxyphényl)-8-(1-phénylméthoxy)éthyl-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydroimidazo[1,2a]pyrazine ;
- la 7-(2-amino-1-oxo-3-thiopropyl)-2-(2-méthyoxyphényl)-8-(phénoxyéthyl)-5,6,7,8-tétrahydro-imidazo[1,2a]pyrazine, ou sa forme dimérique ;
- et la 7-(2-amino-1-oxo-3-thiopropyl)-2-(2-méthoxyphényl)-8-(phénylsulfonyléthyl)-5,6,7,8-tétrahydro-imidazo[1,2a]pyrazine.

Elle a également pour objet lesdits nouveaux produits ou leurs sels pharmaceutiquement acceptables à titre de médicaments, ainsi que leur utilisation pour préparer un médicament destiné à traiter des pathologies qui résultent de la formation de la protéine G hétérotrimérique. En particulier, elle concerne l'utilisation desdits produits pour préparer des médicaments destinés à traiter des maladies liées aux fonctions biologiques ou désordres suivants : odorat, goût, perception de la lumière, neurotransmission, neurodégénérescence, fonctionnement des glandes endocrines et exocrines, régulation autocrine et paracrine, tension artérielle, embryogénèse, prolifération cellulaire bénigne, oncogénèse, infection virale, fonctions immunologiques, diabète, obésité, et maladies prolifératives bénignes et malignes.

Les produits particulièrement préférés pour une utilisation selon l'invention seront donc les suivants :
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine ;
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) ;
- le composé de formule : 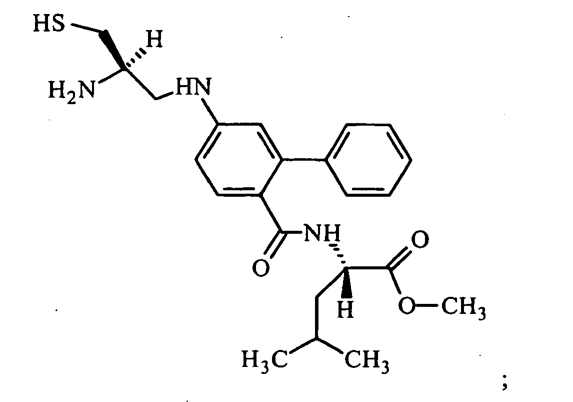
- la 7-(2-amino-1-oxo-3-thiopropyl)-8-(cyclohexylméthyl)-2-(2-méthylphényl)-5,6,7,8-tétrahydroimidazo[1,2a]pyrazine ;
- la 7-(2-amino-1-oxo-3-thiopropyl)-8-(cyclohexylméthyl)-2-phényl-5,6,7,8-tétrahydroimidazo[1,2a]pyrazine ;
- la 7-(2-amino-1-oxo-3-thiopropyl)-2-(2-méthoxyphényl)-8-(phénylméthoxy)méthyl-5,6,7,8-tétrahydroimidazo[1,2a]pyrazine ;
- la 7-(2-amino-1-oxo-3-thiopropyl)-2-(2-méthoxyphényl)-8-(1-phénylméthoxy)éthyl-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydroimidazo[1,2a]pyrazine ;
- la 7-(2-amino-1-oxo-3-thiopropyl)-2-(2-méthyoxyphényl)-8-(phénoxyéthyl)-5,6,7,8-tétrahydroimidazo[1,2a]pyrazine, ou sa forme dimérique ;
- et la 7-(2-ainino-1-oxo-3-thiopropyl)-2-(2-méthoxyphényl)-8-(phénylsulfonyléthyl)-5,6,7,8-tétrahydroimidazo[1,2a]pyrazine ;
- ou un sel pharmaceutiquement acceptable d'un de ces derniers.

De même, l'invention concerne plus particulièrement l'utilisation des composés précédemment cités pour préparer un médicament destiné à traiter le choléra, le Syndrome Immuno-Déficitaire Acquis (SIDA), la diarrhée du voyageur et la puberté précoce familiale masculine.

Les composés de formule générale **(A)** et leur préparation sont décrits dans la demande de brevet WO 97/30053 ou dans les exemples ci-après. La synthèse du composé de formule **(VII)** est décrite dans la partie expérimentale de cette demande.

Les compositions pharmaceutiques comprenant un composé de l'invention peuvent être sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques comprenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection (intramusculaire, sous-cutanée, intraveineuse,etc.), etc. La voie d'administration dépendra bien entendu du type de maladie à traiter.

La dose d'administration envisagée pour un médicament selon l'invention est comprise entre 0,1 mg à 10 g suivant le type de pathologie à traiter.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

### EXEMPLES

### Exemple 1: 7-(2-amino-1-oxo-3-thiopropyl)-8-(cyclohexylméthyl)-2-(2-méthylphényl)-5,6,7,8-tétrahydroimidazo[1,2a]pyrazine : 1

Le composé **1** a été préparé selon le schéma synthétique ci-dessous :

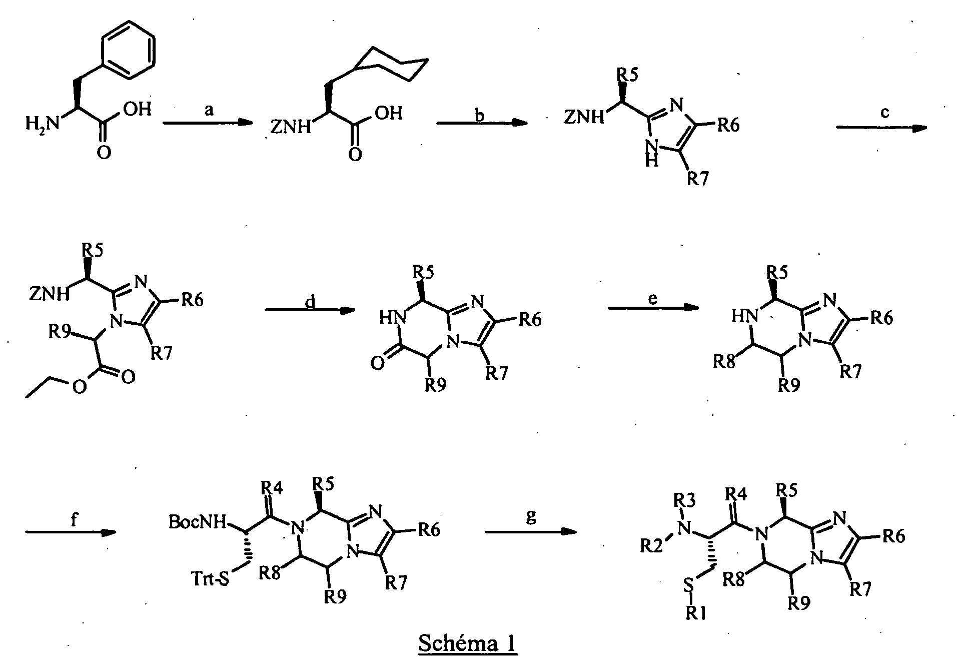

### 1.a) Carbobenryloxy-L-cyclohexylalanine

De la L-phénylalanine (10,0 g ; 60,6 mmol) est combinée avec PtO₂ (430 mg) dans de l'acide acétique (60 ml) et le mélange est hydrogéné sous 20-50 psi H₂ durant une nuit. Une solution aqueuse de HCl à 5% est ajoutée au mélange pour obtenir une solution limpide et l'hydrogénation est poursuivie jusqu'à ce que la consommation d'hydrogène cesse. Le catalyseur est éliminé par filtration et le filtrat concentré sous pression réduite. Le résidu est repris dans du méthanol et de l'eau et le pH ajusté à 4,4 par addition d'une solution de NaOH à 10%. Le produit obtenu est récupéré par filtration et utilisé sans purification supplémentaire.

La L-cyclohexylalanine (60,6mmol) est mise en suspension dans de l'eau (100 ml), du K₂CO₃ (8,36 g ; 60,6 mmol) est ajouté, puis une solution de N-(benzyloxycarbonyloxy)succinimide (15,1 g ; 60,6 mmol) dans CH₃CN (150 ml) et le mélange obtenu est agité vigoureusement durant 45 minutes. Le mélange est concentré pour donner un volume d'environ 100 ml et lavé avec Et₂O (100 ml), puis acidifié avec HCl concentré et extrait avec AcOEt (2x 50 ml). Les phases AcOEt combinées sont séchées sur Na₂SO₄, filtrées et concentrées pour donner une huile claire (17,27 g ; 93 %).

RMN ¹H (DMSO-d6) : 7,5-7,6 (1H, d) ; 7,2-7,5 (5H, m) ; 5,0-5,1 (2H, s) ; 3,9-4,1 (1H, m); 0,7-1,8 (13H, m).

### 1.b) 2-(1-(S)-((phénylméthoxy)carbonyl)-amino-2-(cyclohexyl)méthyl)-4-(2-méthylphenyl)-imidazole

De la Cbz-(L)-cyclohexylalanine (4,58 g ; 15,0 mmol) et du Cs₂CO₃ (2,44 g ; 7,50 mmol) sont placés dans un mélange 2:1 de DMF:H₂O (75 ml). Le mélange obtenu est agité jusqu'à ce qu'il devienne homogène. Les solvants sont éliminés sous pression réduite, le résidu dissous dans du DMF (60 ml) et de la 2-bromo-2'-méthylacétophénone (3,20g ; 15,0 mmol) dans du DMF (30 ml) est ajoutée. Le mélange est agité une nuit durant à température ambiante puis filtré et concentré sous pression réduite. Le céto-ester obtenu est mis en solution dans des xylènes (100 ml) et de l'acétate d'ammonium (19,5 g ; 0,25 mol) est ajouté. Le mélange est chauffé à reflux pendant environ 3 heures avec élimination de AcONH₄ excédentaire et de l'eau libérée par le biais d'un piège de Dean-Stark. Le mélange réactionnel est concentré sous pression réduite, repris dans AcOEt et lavé avec une solution saturée de NaHCO₃ (100 ml) et une solution saturée de NaCl (100 ml). La phase AcOEt est séchée sur Na₂SO₄, filtrée et concentrée sous vide. Le produit brut obtenu est purifié par chromatographie éclair sur gel de silice avec un mélange CHCl₃/MeOH 98/2 comme éluant. Les fractions contenant le produit pur sont combinées et concentrées pour dionner le produit (2,52 g ; 40 %) sous forme d'une mousse légèrement brune qui est utilisée dans l'étape suivante sans purification supplémentaire.

### 1.c) 2-(1-(S)-((phénylméthoxy)carbonyl)-amino-2-(cyclohexyl)méthyl)-1-((2-éthoxy-2-oxo)éthyl)-4-(2-méthylphényl)-imidazole

L'intermédiaire 1.b (2,52 g ; 6,0 mmol) est mis en solution dans du DMF (20 ml) et traité avec K₂CO₃ (1,67g, 12,1 mmol) et du bromoacétate d'éthyle (1,34 ml ; 12,5 mmol) est ajouté. Le mélange obtenu est chauffé à 45° C pendant une heure et demie. Le mélange est dilué avec de l'éther (50 ml) et lavé avec une solution saturée de NaHCO₃ solution (50 ml) puis avec une solution saturée de NaCl (50 ml). La couche éthérée est séchée sur Na₂SO₄, filtrée et concentrée pour donner une huile qui est utilisée dans l'étape suivante sans purification supplémentaire.
Spec. masse : 504,3 MH+.

### 1.d) 8-(cyclohexylméthyl)-6-oxo-2-(2-méthylphényl)-imidazo[1,2-a]pyrazine

L'intermédiaire brut de l'étape 1.c est mis en solution dans de l'acide acétique (50 ml) contenant un catalyseur Pd 10% sur carbone (152 mg), puis hydrogéné sous une pression de 50 psi de H₂ pendant 18 heures à température ambiante. Le catalyseur est éliminé par filtration et le filtrat chauffé à 70° C pendant 2 heures. Le mélange obtenu est concentré sous pression réduite, dissous dans CH₂Cl₂ (100 ml) et lavé avec une solution saturée de.NaHCO₃ (100 ml). La couche du CH₂Cl₂ est séchée sur Na₂SO₄, filtrée et concentrée pour donner une huile visqueuse qui est utilisée dans l'étape suivante sans purification supplémentaire.
Spec. masse : 324,3 MH+.

### 1.e) 8-(cyclohexylméthyl)-2-(2-méthylphényl)-4,5,6,7-tétrahydroimidazo[1, 2-a]pyrazine

L'intermédiaire brut de l'étape 1.c est mis en solution dans du THF (25 ml) et traité à température ambiante par une solution 1*M* de BH3 dans du THF (25 ml) durant une demi-heure puis porté à reflux pendant 1 heure. Le mélange est refroidi par un bain de glace et du HCl 4*N* (40 ml) est additionné goutte à goutte à 0° C. Le mélange est ramené à température ambiante puis porté à reflux durant 1 heure. Le mileu réactionnel est ensuite refroidi, filtré et concentré sous pression réduite. Le résidu est traité avec une solution saturée de NaHCO₃ (50 ml) et extrait avec CH₂Cl₂ (3x 50 ml). Les phases de CH₂Cl₂ sont séchées sur Na₂SO₄, filtrées et concentrées pour donner une huile légèrement brune (1,63 g ; rendement de 87 % par rapport aux étapes 1.c, 1.d et 1.e).
Spec. masse : 310,3 MH+.

### 1.f) 8-(cyclohexylméthyl)- 7-[2-((1,1-diméthyléthoxy)carbonyl)amino)-1-oxo-3-((triphénylméthyl)thio)propyl]- 2-(2-méthylphényl)-4,5,6,7-tétrahydro-imidazo-[1,2a]-pipérazine

Du diisopropylcarbodiimide (908 µl ; 5,80 mmol) et du BocCys(trt)-OH (5,37g ; 11,6 mmol) sont mis en solution dans CH₂Cl₂ (25 ml), le mélange obtenu étant agité durant 45 minutes. De la 8-(cyclohexylméthyl)-2-(2-méthylphényl)-4,5,6,7-tétrahydroimidazo[1,2-a]pyrazine (1,63 g ; 5,27 mmol) est ensuite ajoutée. Le mélange réactionnel est agité durant une nuit à température ambiante. Le solvant est éliminé sous pression réduite et le produit obtenu purifié par chromatographie éclair sur gel de silice avec un mélange CH₂Cl₂/MeOH 98/2 comme éluant. Les fractions pures sont concentrées pour donner une huile visqueuse qui est utilisée dans l'étape suivante sans purification supplémentaire.
Spec. masse : 755,6 MH+.

### 1.g) 7-(2-amino-1-oxo-3-(mercaptopropyl))-8-(cyclohexylméthyl)-2-(2-méthylphényl)-4,5,6,7-tétrahydro-imidazo-[1,2a]-pipérazine: 1 :

L'intermédiaire de l'étape 1.f (3,54 g ; 4,69 mmol) est mis en solution dans de l'acide trifluoroacétique (TFA, 80 ml) contenant du triisopropylsilane (1,92 ml ; 9,38 mmol) et le mélange réactionnel est agité à température ambiante sous azote durant une heure. Le mélange réactionnel est filtré et le filtrat concentré sous pression réduite. Le résidu est extrait par trituration avec une solution aqueuse de TFA à 0,1 % (6x 65 ml) et filtré. Le produit brut est purifié par HPLC préparative sur une colonne C18 en utilisant un gradiant de 0 à 20 % de CH₃CN dans une solution aqueuse de TFA à 0,1 % durant 30 minutes. Les fractions pures de produit sont rassemblées et lyophilisées. Le produit initial est lyophilisé par deux fois à partir d'une solution diluée de HCl pour donner le produit sous forme de son chlorhydrate (740 mg ; 32 %).
Spec. masse : 413,2 MH+.

RMN ¹H (DMSO-d6) : 8,5-9,0 (3H, d, large) ; 7,8-8,0 (1H, s) ; 7,5-7,7 (1H, d) ; 7,2-7,5 (3H, m) ; 5,8-6,1 (1H, m) ; 4,65-4,8 (1H, s) ; 4,5-4,7 (1H, d) ; 4,1-4,4 (2H, m) ; 3,8-4,0 (1H, m) ; 3,2-3,7 (H₂O) ; 2,8-3,1 (2H, m) ; 2,35-2,5 (3H, s) ; 2,0-2,2 (1H, m) ; 1,8-2,05 (2H, m) ; 1,25-1,4 (4H, s large) ; 1,3-0,9 (6H, m).

### Exempte 2 : 7-(2-amino-1-oxo-3-thiopropyl)-8-(cyclohexylméthyl)-2-phényl-5,6,7,8-tétrahydroimidazo[1,2a]pyrazine : 2 :

Le composé **2** est préparé selon le schéma 1, étapes b à g, selon une méthode analogue à celle de l'exemple 1, la 2-bromoacétophénone remplaçant la 2-bromo-2'-méthylacétophénone dans l'étape b.
Spec. masse : 399,2 MH+.

RMN ¹H (DMSO-d6) : 8,5-8,9 (3H, d large) ; 8,0-8,2 (1H, s) ; 7,8-8,0 (2H, d) ; 7,45-7,56 (2H, t) ; 7,35-7,5 (1H, t) ; 5,9-6,05 (1H, s large) ; 4,65-4,8 (1H, s) ; 4,5-4,65 (1H, d) ; 4,1-4,35 (2H, m) ; 3,8-4,0 (1H, m) ; 3,2-3,8 (H₂O) ; 3,25-3,4 (1H, t) ; 2,8-3,05 (2H, m) ; 2,05-2,2 (1H, d) ; 1,85-2,05 (2H, t) ; 1,55-1,75 (4H, s large) ; 1,15-1,3 (1H, s large) ; 1,2-0,9 (5H, m).

### Exemple 3 : 7-(2-amino-1-oxo-3-thiopropyl)-2-(2-méthoxyphényl)-8-(phénylméthoxy)méthyl-5,6,7,8-tétrahydroimidazo[1,2a]pyrazine : 3 :

Le composé **3** est préparé selon le schéma 1, étapes b à g, selon une méthode analogue à celle de l'exemple 1, le Boc-(L)-Ser(Bzl)-OH remplaçant la Cbz-(L)-cyclohexylalanine dans l'étape b et l'étape d étant remplacée par une déprotection utilisant le TFA et iPr₃SiH selon une méthode analogue à la réaction 1.g. Le produit est obtenu sous forme d'un couple de diastéréoisomères dans une proportion 2:3.
Spec. masse : 453,2 MH+.

Les temps de rétention pour les diastréoisomères sont de 6,58 et 7,07 minutes respectivement dans le système de HPLC suivant :
- Eluant :: 30-50% CH₃CN / 0.1% TFA
- Durée d'élution :: 24 minutes
- Détection :: 254 nm
- Colonne :: protéine Vydac et peptide C18

### Exemple 4 : 7-(2-amino-1-oxo-3-thiopropyl)-2-(2-méthoxyphényl)-8-(1-phénylméthoxy)éthyl-5,6,7,8-tétrahydroimidazo[1,2a]pyrazine : 4 :

Le composé **4** est préparé selon le schéma 1, étapes b à g, selon une méthode analogue à celle de l'exemple 3, le Boc-(L)-Thr(Bzl)-OH remplaçant le Boc-(L)-Ser(Bzl)-OH dans l'étape b.
Spec. masse : 467,3 MH+.

RMN ¹H (DMSO-d6) : 8,5-8,9 (3H, d, large) ; 8,0-8,1 (1H, s) ; 7,95-8,1 (2H, d) ; 7,4-7,5 (1H, t) ; 7,15-7,3 (1H, d) ; 7,0-7,2 (3H, m) ; 6,9-7,05 (2H, m) ; 5,85-5,95 (1H, d) ; 4,75-4,85 (1H, s large) ; 4,65-4,8 (1H, s large) ; 4,35-4,65 (3H, m) ; 4,1-4,25 (2H, q) ; 3,9-4,0 (3H, s) ; 2,8-3,1 (2H, m) ; 1,2-1,4 (3H, d).

### Exemple 5 : 7-(2-amino-1-oxo-3-thiopropyl)-2-(2-méthoxyphényl)-8-(phénoxyéthyl)-5,6,7,8-tétrahydroimidazo[1,2a]pyrazine : 5 :

Le composé **5** a été préparé selon le schéma synthétique ci-après :

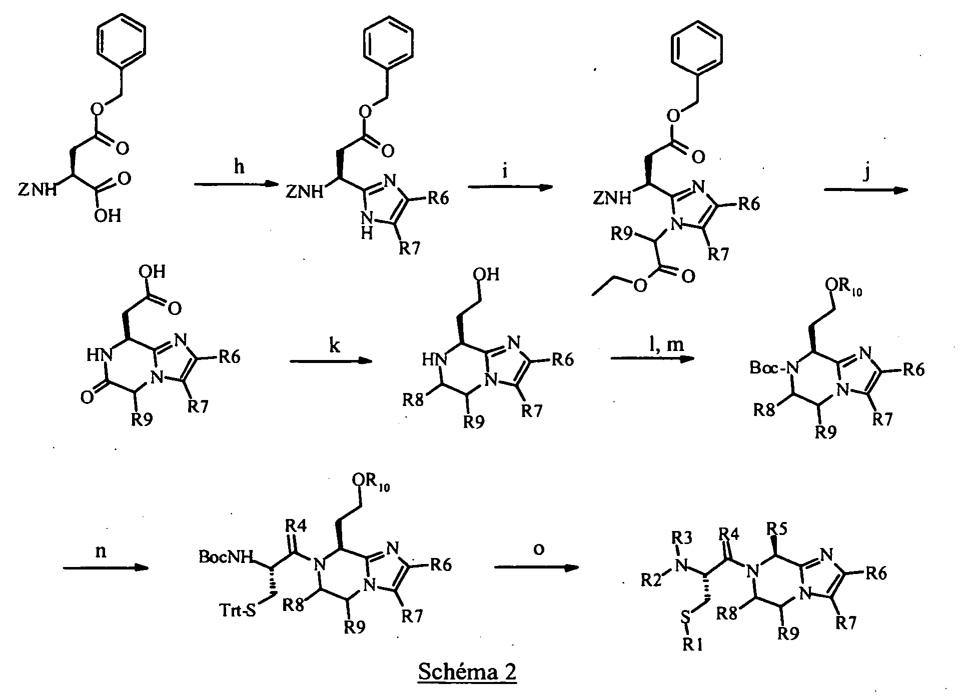

### 5.h) 2-(1-(S)-((phénylméthoxy)carbonyl)-amino-2-(2-oxo-2-(phénylméthoxy)éthyl)-4-(2-méthoxyphényl)-imidazole

Du Cbz-(L)-Asp(Obzl)-OH (5,00 g ; 14,0 mmol) et du Cs₂CO₃ (2,28 g ; 7,00 mmol) sont mélangés dans un mélange 1:1 1 de DMF:H₂O (75 ml). Le mélange obtenu est agité jusqu'à ce qu'il devienne homogène. Les solvants sont éliminés sous pression réduite, le résidu dissous dans du DMF (60 ml) et de la 2-bromo-2'-méthoxyacétophénone (3,21 g ; 14.0 mmol) dans du DMF (30 ml) est ajoutée. Le mélange obtenu est agité durant u!ne demi-heure à température ambiante puis filtré et concentré sous pression réduite. Le céto-ester obtenu est trituré avec un mélange 1:1 de Et₂O:hexanes (2x 40 ml) puis mis en suspension dans des xylènes (100 ml). De l'acétate d'ammonium (17,5 g ; 0,23 mol) est ajouté et le mélange est chauffé à reflux pendant environ une heure et demie avec élimination de AcONH₄ en excès et de l'eau libérée par le biais d'un piège de Dean-Stark. Le milieu réactionnel est lavé avec une solution saturée de NaHCO₃ (50 ml), séché sur Na₂SO₄, filtré et concentré sous vide pour donner 6,66g (98 %) du produit désiré.
Spec. masse : 486,3 (MH+).

### 5.i) 2-(I-(S)-((phénylméthoxy)carbonyl)-amino-2-((2-phénylméthoxy-2-oxo)éthyl)-1-((2-éthoxy-2-oxo)éthyl)-4-(2-méthoxyphényl)-imidazole

L'intermédiaire 5.i est préparé selon une méthode analogue à celle de l'étape 1.c.
Spec. masse : 572,3 MH+.

### 5.j) acide (6-oxo-2-(2-méthoxyphényl)-5,6,7,8-tétrahydro-imidazo[1,2-a]pyrazin-8-yl)-acétique

L'intermédiaire 5.j est préparé selon une méthode analogue à celle de l'étape l.d.
Spec. masse : 302,2 MH+.

RMN ¹H (DMSO-d6) : 8,35-8,5 (1H, d, large) ; 8,0-8,1 (1H, dd) ; 7,45-7,55 (1H, s) ; 7,15-7,25 (1H, m) ; 7,0-7,1 (1H, m) ; 6,9-7,0 (1H, m) ; 4,85-5,0 (1H, s large) ; 4,55-4,75 (2H, q) ; 3,85-3,95 (3H, s) ; 2,8-2,95 (2H, d).

### 5.k) 8-hydroxyéthyl-2-(2-méthoxyphényl)-5,6,7,8-tétrahydro-imidazo[1,2-a]pyrazine

L'intermédiaire 5.k est préparé selon une méthode analogue à celle de l'étape 1.e, excepté le fait qu'une proportion molaire de 6/9 de BH₃ par rapport au substrat est utilisée.
Spec. masse : 274,3 MH+.

### 5.1) 7-((1,1-diméthyléthoxy)carbonyl)-8-hydroxyéthyl-2-(2-méthyoxyphényl) - 5,6,7,8-tétrahydro-imidazo[1,2-a]pyrazine

L'intermédiaire 5.k (1,36 g ; 5,0 mmol) est mis en suspension dans H₂O (5 ml) et un mélange de di-t-butyldicarbonate (1,20 g ; 5,5 mmol) dans du p-dioxane (10 ml) est ajouté. Le mileu réactionnel est agité vigoureusement et maintenu à pH 8,0-8,4 par addition goutte à goutte d'une solution 2,5*N* de NaOH jusqu'à ce que la réaction soit terminée (suivi de la réaction par CCM sur gel de silice, éluant AcOEt:hexanes 3:2). Le produit brut est purifié par chromatographie éclair sur gel de silice avec un mélange AcOEt:hexanes 3:2 comme éluant (système Biotage, colonnes pré-remplies 4 x 15 cm). Les fractions contenant le produit sont combinées et concentrées sous vide pour donner une mousse blanche (1,60 g ; 86 %).
Spec. masse : 374,3 MH+.

RMN ¹H (DMSO-d6) : 7,95-8,05 (1H, d,d) ; 7,45-7,55 (1H, s) ; 7,10-7,25 (1H, m) ; 7,0-7,1 (1H, m) ; 6,9-7,05 (1H, m) ; 5,05-5,15 (1H, t) ; 4,25-4,35 (1H, t) ; 4,05-4,2 (1H, s large) ; 4,0-4,1 (1H m) ; 3,9-4,0 (3H, s) ; 3,9-4,0 (1H, m) ; 3,25-3,35 (2H, m) ; 1,9-2,1 (2H, m) ; 1;15-1,25 (9H, s).

### 5.m) 7-((1,1-diméthyléthoxy)carbonyl)-8-phénoxyéthyl-2-(2-méthoxyphényl) -5,6,7,8-tétrahydro-imidazo[1,2-a]pyrazine

L'intermédiaire 5.1 (746 mg ; 2,00 mmol) est dissous dans du THF (10 ml) contenant de la triphénylphosphine (550 mg ; 2,1 mmol) et du phénol (198 mg; 2,1 mmol). Le mélange est refroidi à 0 °C sous azote et du diéthylazodicarboxylate (330 µl ; 2,1 mmol) est ajouté goutte à goutte durant 10 minutes. Le mélange réactionnel est ensuite agité durant 2 heures à température ambiante. Le milieu réactionnel est ensuite refroidi à nouveau à 0 °C et de la triphénylphosphine (275 mg ; 1,05 mmol) et du phénol (99 mg ; 1,05 mmol) sont ajoutés. On ajoute ensuite du diéthylazodicarboxylate (166 µl ; 1,05 mmol) goutte à goutte durant 10 minutes puis le mélange est agité encore pendant 1 heure à température ambiante. Les solvants sont éliminés sous pression réduite et le produit brut est purifié par chromatographie éclair sur gel de silice avec un mélange AcOEt:hexanes 3:2 comme éluant. Les fractions contenant le produit sont combinées et concentrées sous vide. Après recristallisation dans AcOEt et les hexanes, on obtient le produit désiré sous forme d'un solide blanc (863 mg ; 96 %).
Spec. masse : 450,4 MH+.

### 5.n) 7-(2-(((1,1-diméthyléthoxy)carbonyl)amino)-1-oxo-3-((triphénylméthyl) thio)propyl)- 2-(2-méthoxyphényl)-8-(phénoxyéthyl)-4,5,6,7-tétrahydro-imidazo-[1,2a]-pipérazine

L'intermédiaire 5.m (850 mg; 1,89 mmol) est traité avec un mélange de TFA (10 ml) contenant iPr₃SiH (387µl, 1.89mmol) à température ambiante pendant 20 min. Les solvants sont éliminés sous pression réduite et le produit brut est réparti entre AcOEt (15 ml) et une solution saturée de NaHCO₃ (15 ml). La phase AcOEt est séchée sur Na₂SO₄, filtrée et concentrée sous pression réduite. Le produit déprotégé est couplé à Boc-(L)-Cys(Trt)-OH selon une méthode analogue à celle de l'étape 1.f (1,26 g ; 84 %).

### 5.o) 7-(2-amino-1-oxo-3-thiopropyl)-2-(2-méthoxyphényl)-8-(phénoxyéthyl)-4,5,6,7-tétrahydro-imidazo-[1,2a]-pipérazine

Le produit **5** est préparé à partir de l'intermédiaire 5.n selon une méthode analogue à celle de l'étape 1.g.
Spec. masse : 274,3 MH+.

RMN ¹H (DMSO-d6 à 90 °C) : 8,5-9,2 (3H, s, large) ; 7,95-8,1 (1H, d) ; 7,85-8,0 (1H, s) ; 7,35-7,5 (1H, m) ; 7,15-7,35 (3H, m) ; 7,0-7,15 (1H, t) ; 6,85-7,0 (3H, m) ; 5,9-6,1 (1H, s, large) ; 4,5-4,8 (2H, m, large) ; 4,15-4,45 (3H, m, large) ; 3,9-4,0 (3H, s) ; 3,75-4,0 (1H, m, large) ; 2,8-3,05 (2H, m, large) ; 2,55-2,75 (2H, m, large).

### Exemple 6 : 7-(2-amino-1-oxo-3-thiopropyl)-2-(2-méthoxyphényl)-8-(phénoxyéthyl)-5,6,7,8-tétrahydro-imidazo[1,2a]pyrazine, dimère : 6 :

Le composé 5.o (467 mg ; 0,687 mmol) est mis en solution dans H₂O (25 ml) et le pH de la solution est ajusté à 7,2 par addition d'une solution aqueuse diluée de NH₄OH. De l'acétonitrile est ajouté pour donner une solution limpide et le mélange est agité à température ambiante durant une nuit. Le produit brut est purifié par HPLC préparative sur une colonne C 18 en utilisant un gradient de 15 à 40 % CH₃CN dans du TFA à 0,1 % sur une durée de 50 minutes. Les fractions pures de produit sont rassemblées et lyophilisées. Le produit initial est lyophilisé par deux fois à partir d'une solution diluée de HCl pour donner le produit sous forme de son chlorhydrate (161 mg ; 45 %).
Spec. masse : 903,5 MH+.

RMN ¹H (DMSO-d6 à 90° C) : 8,7-9,3 (3H, s large) ; 7,95-8,1 (1H, d) ; 7,85-8,0 (1H, s) ; 7,3-7,5 (1H, t) ; 7,1-7,3 (3H, m) ; 7,0-7,15 (1H, t) ; 6,8-7,0 (3H, m) ; 5,85-6,1 (1H, s large) ; 4,7-4,9 (1H, s large) ; 4,45-4,7 (1H, m large) ; 4,1-4,5 (4H, m large) ; 3,85-4,0 (4H, s) ; 3,3-3,5 (2H, m large) ; 2,5-2,8 (2H, m large).

### Exemple 7 : 7-(2-amino-1-oxo-3-thiopropyl)-2-(2-méthoxyphényl)-8-(phénylsulfonyléthyl)-5,6,7,8-tétrahydroimidazo[1,2a]pyrazine : 7 :

Le composé **7** a été préparé selon le schéma synthétique ci-dessous :

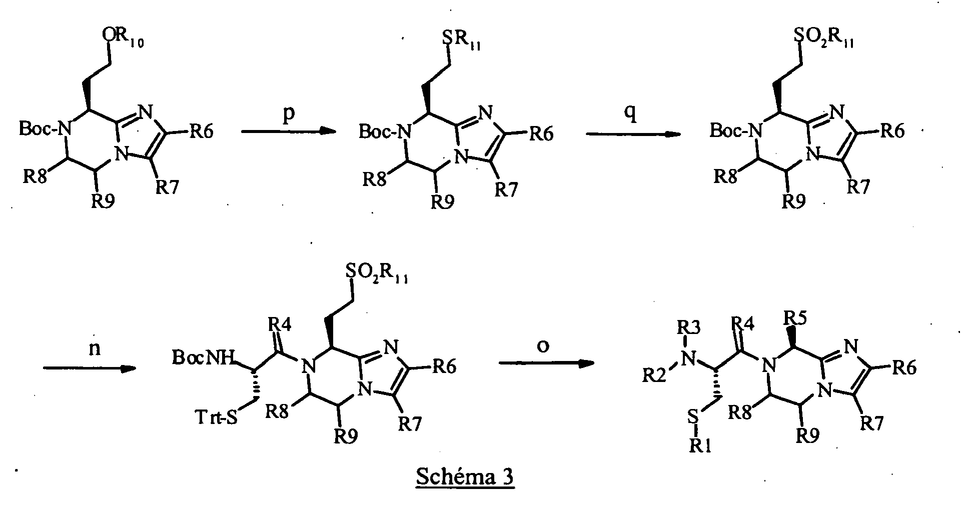

### 7.p) 7-((1,1-diméthyléthoxy)carbonyl)-2-(2-méthoxyphényl)-8-(phénylthioéthyl)-5,6,7,8-tétrahydro-imidazo[1,2a]pyrazine

De l'intermédiaire 5.1 (1,23 g; 3,30 mmol), de la tri-n-butylphosphine (1,64 ml ; 6,60 mmol), et du phényldisulfide (1,44 g ; 6,60 mmol) sont mélangés dans du THF (10 ml). Le mélange est agité à température ambiante sous argon durant 4 heures. Les solvants sont éliminés sous pression réduite et le produit brut est purifié par chromatographie éclair sur gel de silice avec un mélange AcOEt:hexanes 1:1 comme éluant. Les fractions contenant le produit sont combinées et concentrées sous vide pour donner le produit sous forme d'une mousse blanche (1,43 g ; 93 %).
Spec. masse : 466,3 MH+.

### 7.q) 7-((1,1-dïméthyléthoxy)carbonyl)-2-(2-méthoxyphényl)-8-(phénylsulfonyléthyl)-5,6,7,8-tétrahydro-imidazo[1,2a]pyrazine

L'intermédiaire 7.p (650 mg; 1,40 mmol) est dissous dans du CH₂Cl₂ (10 ml) et de l'acide 3-chloroperoxybenzoïque (483 mg ; 2,80 mmol) est ajouté en plusieurs portions sur une période de 10 minutes. Le mélange est versé sur une colonne de silice et élué avec un mélange hexanes:AcOEt 7:3, puis un mélange hexanes:AcOEt 1:1 pour donner le produit pur (220 mg ; 32 %).
Spec. masse : 498,3 MH+.

RMN ¹H (DMSO-d6 à 30 °C) : 7,9-8,0 (3H, m) ; 7,7-7,85 (1H, m) ; 7,6-7,75 (2H, m) ; 7,45-7,55 (1H,s) ; 7,15-7,25 (1H, m) ; 6,9-7,1 (2H, m) ; 5,1-5,25 (1H, t) ; 4,1-4,3 (1 H, d large) ; 4,0-4,15 (1H, m) ; 3,8-4,0 (1H, m) ; 3,85-3,95 (3H, s) ; 3,6-3,8 (1H, m) ; 3,4-3,6 (1H, m) ; 3,2-3,4 (H₂O plus un signal flou) ; 1,9-2,3 (2H, m) ; 1,3-1,5 (9H, s).

### Etape 7.n)

L'étape 7.n est réalisée selon une méthode analogue à l'étape 5.n. Le produit brut est engagé sans purification complémentaire dans l'étape suivante.

### Etape 7.o)

L'étape 7.o est réalisée selon une méthode analogue à l'étape 5.o.
Spec. masse : 501,3 MH+.

### Préparation du composé de formule (VII):

Ce composé, proche de ceux décrits dans la demande de brevet PCT WO 97/30053, peut être préparé selon le schéma synthétique suivant :

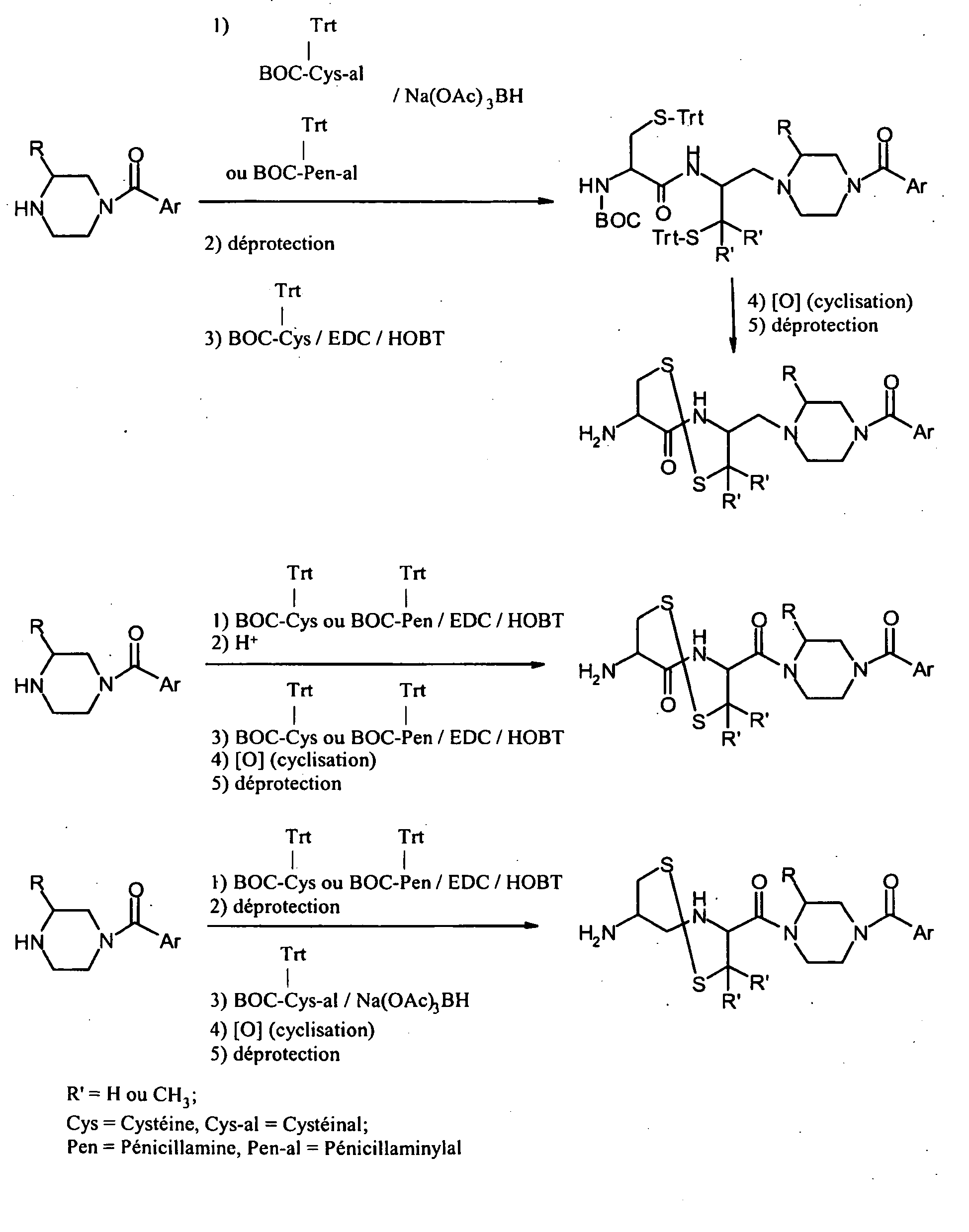

### PARTIE PHARMACOLOGIOUE

Afin d'illustrer l'utilité de l'invention, on étudiera l'effet d'un traitement d'une lignée de cellules humaines MCF-7 par les composés suivants :
- disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine, désigné dans cette partie comme composé **(I) ;**
- disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle), désigné dans cette partie comme composé **(II) ;**
- 7-(2-amino-1-oxo-3-thiopropyl)-8-(cyclohexylméthyl)-2-(2-méthylphényl)-5,6,7,8-tétrahydroimidazo[1,2a]pyrazine, désigné dans cette partie comme composé **(III) ;**
- le composé de formule : 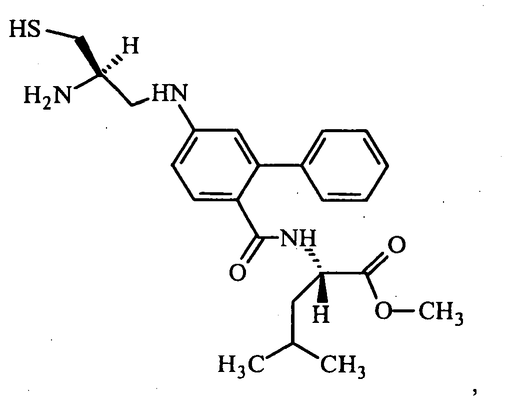 désigné dans cette partie comme composé **(IV) ;**
- la 7-(2-amino-1-oxo-3-thiopropyl)-8-butyl-2-(2-méthoxyphényl)-5,6,7,8-tétrahydroimidazo[1,2a]pyrazine, désigné dans cette partie comme composé **(V) ;**
- le disulfure de bis-1,1'-[7-(2-amino-1-oxo-3-thiopropyl)-2-(méthoxyphényl)-8-(1-méthylpropyl)--5,6,7,8-tétrahydroimidazo[1,2a]pyrazine], désigné dans cette partie comme composé **(VI) ;**
- le composé de formule : 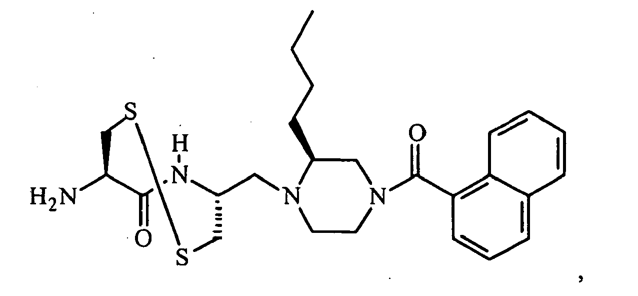 désigné dans cette partie comme composé **(VII) ;**

### Procédures

### Lignée cellulaire

Les lignées cellulaires MCF-7 (cellules pleurales humaines, cancer du sein) ont été acquises auprès de American Tissue Culture Collection (Rockville, Maryland, USA).

### Mesure de la quantité intracellulaire d'AMP cyclique pour les cellules MCF-7

Des cellules MCF-7 (2.10⁴ cellules/puits) ensemencées en plaques de 24 puits, sont mises en culture durant 5 jours dans le milieu de Eagle modifié par Dulbecco (Gibco-Brl, Cergy-Pontoise, France) complété avec 10% de sérum foetal de veau inactivé par chauffage (Gibco-Brl, Cergy-Pontoise, France ), 50000 unités/l de pénicilline et 50 mg/l streptomycine (Gibco-Brl, Cergy-Pontoise, France), et 2 mM de glutamine (Gibco-Brl, Cergy-Pontoise, France). Le milieu de culture est remplacé après deux lavages avec un milieu sans sérum complété ou non avec les agents spécifiés pour un temps indiqué dans les différentes figures. Des agents activant la production d'AMPcycliqueyclique sont ensuite ajoutés à 37 °C. La réaction est stoppée au bout de 30 minutes par suppression du milieu et addition rapide de 100 µl de solution HCl 0,1N. Ces extraits sont congelés à -80 °C jusqu'à ce qu'ils soient utilisés. La concentration d' AMPc est mesurée en utilisant un kit commercial de mesure (référence NEK033 de NEN, Les Ulis, France) en suivant les instructions du fabricant. La radioactivité est déterminée par un compteur Gamma (Gamma Maser-1277, LKB, Turku, Finland ).

### Mesure de la prolifération cellulaire in vitro

Les cellules MCF-7 (3000 cellules/puits) sont cultivées en plaques 96 puits dans 80 µl de milieu de Eagle modifié par Dulbecco (Gibco-Brl, Cergy-Pontoise, France) complété avec 10% de sérum foetal de veau inactivé par chauffage (Gibco-Brl, Cergy-Pontoise, France), 50000 unités/l de pénicilline et 50 mg/l streptomycine (Gibco-Brl, Cergy-Pontoise, France), et 2 mM de glutamine (Gibco-Brl, Cergy-Pontoise, France) ont été ensemencées sur une plaque de 96 puits au jour 0. Les cellules ont été traitées au jour 1 pendant 96 heures avec des concentrations croissantes allant jusqu'à 50 µM de chacun des composés à tester. A la fin de cette période, la quantification de la prolifération cellulaire est évaluée par test colorimétrique en se basant sur le clivage du sel de tétrazolium WST1 par les déhydrogénases mitochondriales dans les cellules viables conduisant à la formation de formazan (Boehringer Mannheim, Meylan, France). Ces tests sont effectués en double avec 8 déterminations par concentration testée. Pour chaque composé à tester, les valeurs incluses dans la partie linéaire de la sigmoïde ont été retenues pour une analyse en régression linéaire et utilisées pour estimer la concentration inhibitrice (CI₅₀).

### Mesure de l'activité de la MAP kinase

Des cellules MCF7 (5.105 cellules/puits) sont cultivées en plaques 6 puits dans le milieu de Eagle modifié par Dulbecco (Gibco-Brl, Cergy-Pontoise, France) complété avec 10 % de sérum de veau foetal inactivé par la chaleur (Gibco-Brl, Cergy-Pontoise, France), un mélange d'antibiotiques : 50000 unités/l de pénicilline et 50 mg/l de streptomycine (Gibco-Brl, Cergy-Pontoise, France) et 2 mM de glutamine (Gibco-Brl, Cergy-Pontoise, France).Après 24 heures de culture, les cellules sont incubées pendant 48 heures dans du milieu ne contenant pas de sérum pour amener les cellules à l'état de repos. Les cellules sont alors traitées pendant 1 heure soit par le composé I soit par le PD98059 (Calbiochem,France Biochem, Meudon, France), un inhibiteur spécifique de l'activation de la MAP kinase. Les cellules sont ensuite stimulées (ou non) pendant 5 minutes par 12.5 ng/ml de facteur de croissance épidermique (EGF). La réaction est stoppée par deux lavages avec du PBS(Gibco-Brl, Cergy-Pontoise, France), à 4° C ne contenant ni calcium ni magnésium et par addition de 150 µl de tampon de lyse à 4° C dont la composition est la suivante: 10 mM de tris, 150 mM de NaCl, 2 mM d'EGTA, 2 mM de dithiothreitol, 1 mM de PMSF,2 mM d'orthovanadate, 10µg/ml de leupeptine et 10 µg/ml d'aprotinine. Un dosage des protéines contenues dans les extraits est réalisé par la méthode de Bradford (réactifs Biorad,Ivry-Sur-Seine, France). Ces extraits sont congelés à -80 °C jusqu'à ce qu'ils soient utilisés. L'activité de la MAP kinase est mesurée à l'aide d'un kit commercial de mesure (référence RPN 84, Amersham, Life Science, Les Ulis, France) en suivant les instructions du fabricant. La radioactivité est déterminée à l'aide d'un compteur à scintillation Packard (Tricarb 5000CA).

### Matériel

Le peptide vaso-intestinal (VIP) a été acquis chez Bachem (Voisins le Bretonneux, France). La toxine cholérique, la forskoline, l'isoprotérénol, la prostaglandine E2 et le PD 98059 ont été acquis chez Calbiochem (France Biochem, Meodon, France). Les composés de formules **(I), (II), (III), (IV), (V), (VI)** et **(VII)** ont été fournis par Biomeasure Inc. (Milford, MA, USA). Tous ces composés ont été utilisés en suivant les recommandations de leurs fabricants.

### Résultats

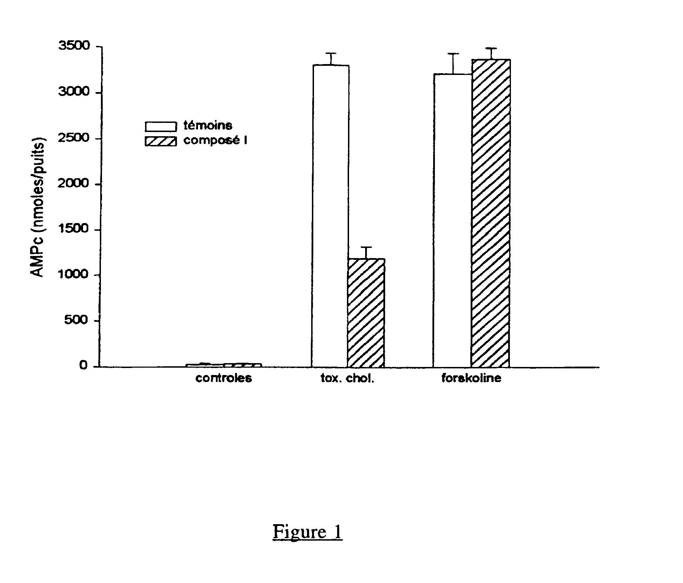

La figure 1 montre que l'activation de l'adénylate cyclase par la toxine cholérique (200 ng/ml) ou par la forskoline (10 µM) conduit à une élévation très importante du taux d'AMP cyclique. Un prétraitement des cellules pendant 30 minutes avec 30 µM de composé **(I)** ne modifie pas la production d'AMP cyclique induite par l'activateur direct de l'adénylate cyclase, la forskoline. En revanche, la production d'AMP cyclique stimulée par l'activateur direct de la sous-unité, la toxine cholérique, est fortement inhibée par le composé **(I).** Ceci montre que l'adénylate cyclase, elle-même, n'est pas modifiée par lé composé **(I)** et que celui-ci empêche la formation du complexe hétérotrimérique.

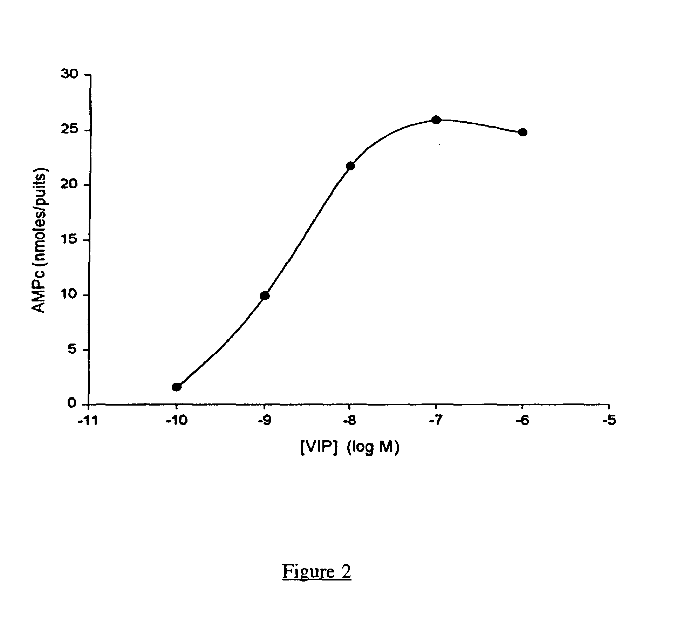

Le VIP a été présenté comme un ligand extra-cellulaire d'un récepteur couplé à la protéine G qui stimule la synthèse d'AMP cyclique dans des cellules humaines de cancer du sein. La figure 2 montre que le traitement par le VIP de cellules humaines de cancer du sein MCF-7 augmente la quantité intracellulaire d'AMP cyclique de façon concentration-dépendante. La concentration de VIP de 10 nM qui offre une production d'AMP cyclique quasi-optimale sera utilisée pour les tests suivants. Cette concentration est en accord avec les données déjà publiées concernant la lignée de cellules humaines de cancer du sein T47D.

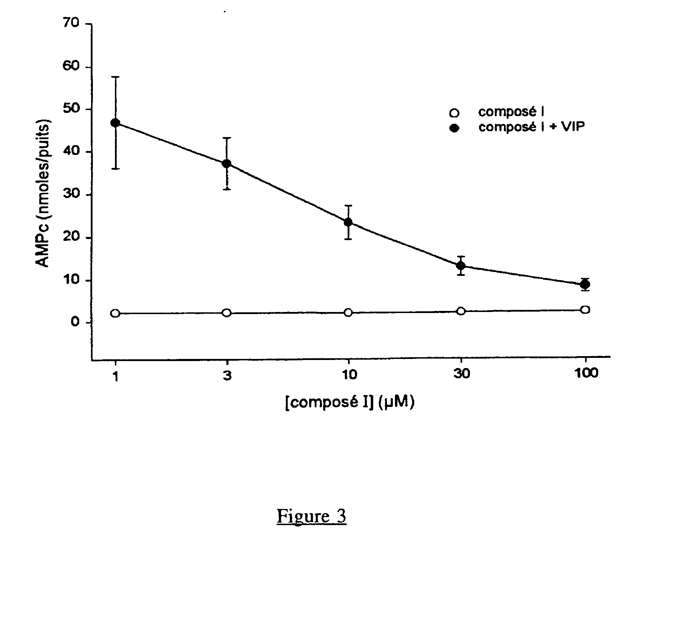

La figure 3 montre qu'un prétraitement de 30 minutes des cellules MCF-7 issues de cultures *in vitro* par le composé de formule **(I)** est suffisant pour inhiber de façon dépendante de la concentration, l'accumulation d'AMP cyclique stimulée par le VIP. Une inhibition presque complète a été obtenue à une concentration de 100 µM du composé de formule **(I).** Ces résultats montrent qu'un traitement par le composé **(I)** est suffisant pour bloquer la transduction du signal dont la voie utilise les protéines G hétérotrimériques comme médiateurs.

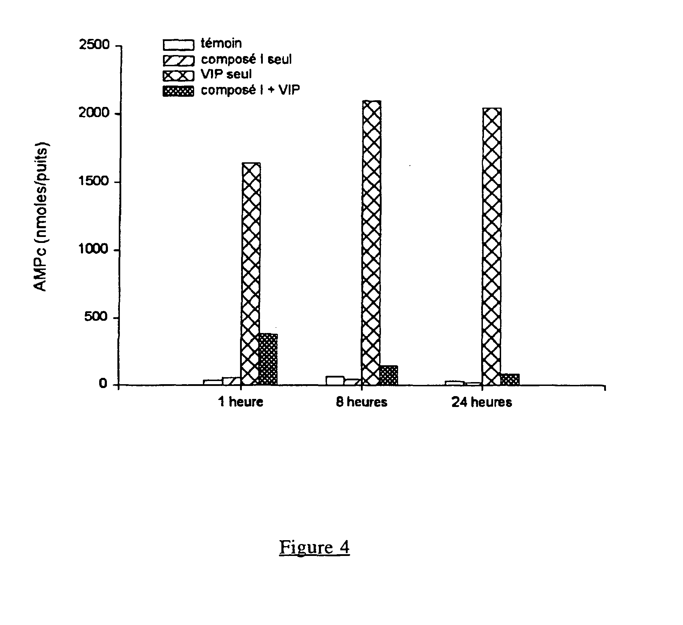

La figure 4 montre qu'un traitement d'une heure par le composé de formule **(I)** est suffisant pour modifier la réponse au VIP. Des traitements d'une durée plus longue (8 heures et 24 heures) continuent d'inhiber la production d'AMP cyclique mais le principal effet est obtenu très rapidement.

Le composé **(I)** est aussi capable d'inhiber la formation d'AMP cyclique induite par d'autres agents qui stimulent les récepteurs à sept domaines transmembranaires. Dans les cellules MCF7, par exemple, l'activité de l'adénylate cyclase très fortement augmentée par la prostaglandine E₂ est inhibée par un traitement de 30 minutes par le composé **(I).** Ceci suggère que le traitement des cellules par le composé **(I)** modifie la forme hétérotrimérique des protéines G en dissociant la sous-unité du dimère β/γ.

L'inhibition de la stimulation par le VIP n'est pas restreinte à des composés de structure analogue à celle du composé de formule **(I)** Comme le montre le tableau I, les composés **(II), (III), (IV), (V), (VI)** et **(VII)** testés dans le même modèle, sont aussi capables de réduire la quantité d'AMP cyclique induite par le VIP.

L'ensemble de ces résultats suggèrent que les composés testés modulent l'activité de l'adénylate cyclase en modifiant la forme hétérotrimérique des protéines G. Or, on sait que les dimères β/γ peuvent activer directement des effecteurs conduisant à l'activation de kinases régulées par des signaux extracellulaires (ERKs) ou MAP kinases.

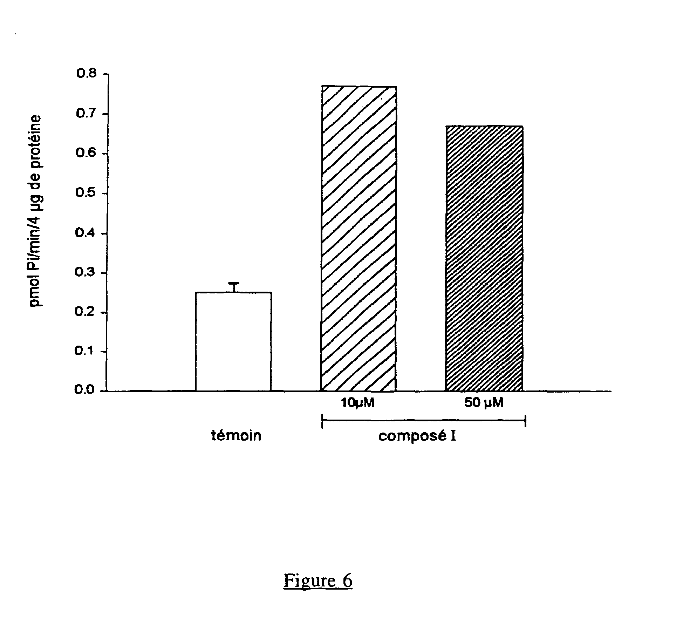

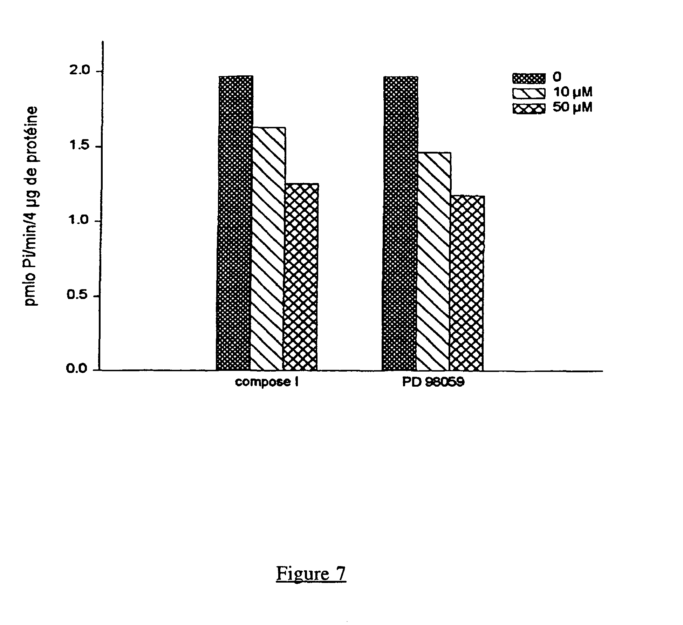

La figure 6 montre qu'un traitement des cellules pendant 1 heure par le composé **(I)** double l'activité basale de la MAP kinase . Ceci suggère qu'en empêchant la formation du complexe hétérotrimèrique, le composé **(I)** libère l'hétérodimère - qui, lui, reste lié à la membrane et active la voie de ras. En revanche, la figure 7 montre qu'après une stimulation de 5 minutes de la MAP kinase par le facteur de croissance EGF, l'activité de l'enzyme est augmentée d'environ 7 fois. Un pré-taitement pendant 1 heure des cellules soit par le composé **(I)** soit par le PD98059, un inhibiteur spécifique de l'activation de la MAP kinase, réduit de 2 fois l'activité de la MAP kinase. Ces résultats suggèrent que le composé **(I)** stimule à l'état basal la voie de ras et inhibe cette même voie si celle-ci est stimulée expliquant ainsi son effet antiprolifératif.
Le tableau II montre en effet que les composés **(I), (II), (III)** et **(IV)** sont capables d'inhiber la prolifération *in vitro* des cellules tumorales humaines MCF7.

| Composé | Inhibition à 30µM |
|---|---|
| **(I)** | 86% |
| **(II)** | 71 % |
| **(III)** | 59 % |
| **(IV)** | 52 % |
| **(V)** | 68 % |
| **(VI)** | 52 % |
| **(VII)** | 65 % |

### Tableau 1

### Effets des composés I, II, III et IV incubés pendant 30 minutes sur la production d'AMP cyclique stimulée par le VIP dans les cellules MCF7.

Les cellules sont incubées pendant 30 minutes en présence ou non des composés I, II, III et IV (30 µM) qui sont stimulées ensuite par 10⁻⁸ M de VIP. La quantification d'AMP cyclique est déterminée par dosage radio-immunologique. Les données représentent la moyenne ± ESM (n=5 pour le témoin et n=1 pour les différents composés ).

| Composé testé | CI₅₀ (µM) |
|---|---|
| composé I | 9,4 |
| composé II | 15,0 |
| composé III | 16,1 |
| composé IV | 34,6 |

### Tableau II

### Inhibition de la croissance in vitro des cellules MCF7 par les composés I, II, III et IV.

Les résultats des CI₅₀ sont exprimés en µM et représentent la moyenne de 2 expériences.

### FIGURES

**Figure 1** **: Effet du composé I sur la production d'AMP cyclique stimulée par la toxine cholérique ou la forskoline dans les cellules MCF7**
   Les cellules sont incubées pendant 30 minutes en présence ou non du composé 1 (30 µM) et stimulées ensuite soit par la toxine cholérique (200 ng/ml) pendant 90 minutes soit par la forskoline (10⁻⁵ M) pendant 30 minutes. La quantification d'AMP cyclique est déterminée par dosage radio-immunologique. Les données représentent la moyenne ± ESM (n=3)
**Figure 2** **: Effet de concentrations croissantes de VIP sur la production d'AMP cyclique dans les cellules MCF7**
   Les cellules sont stimulées pendant 30 minutes par le VIP aux concentrations indiquées. La quantification d'AMP cyclique est déterminée par dosage radio-immunologique.
**Figure 3** **: Effet de différentes concentrations de composé I sur la production d'AMP cyclique stimulée par le VIP dans les cellules MCF7**
   Les cellules sont incubées pendant 24 heures en présence de concentrations croissantes du composé I et stimulées ensuite pendant 30 minutes par 10⁻⁸ M de VIP. La quantification d'AMP cyclique est déterminée par dosage radio-immunologique. Les données représentent la moyenne ± ESM (n = 3).
**Figure 4** **: Effet du temps d'incubation des cellules MCF7 en présence du composé I sur la production d'AMP cyclique stimulée par le VIP**
   Les cellules sont incubées pendant 1, 8 ou 24 heures en présence ou non du composé I (30 µM) et stimulées ensuite par 10⁻⁸ M de VIP. La quantification d'AMP cyclique est déterminée par dosage radio-immunologique. Les données représentent la moyenne ± ESM (n=1).

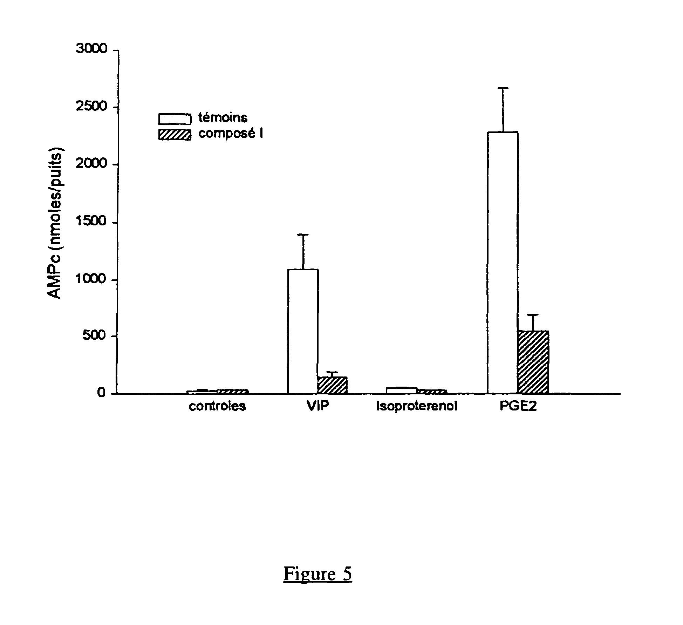

**Figure 5** **: Effet du composé I sur la production d'AMP cyclique stimulée par le VIP, l'isoprotérénol ou la prostaglandine E₂ dans les cellules MCF7**
   Les cellules sont incubées pendant 30 minutes en présence ou non du composé I (30 µM) et stimulées ensuite pendant 30 minutes soit par le VIP (10⁻⁸ M), soit par l'isoprotérénol (10⁻⁶ M), soit par la PGE₂ (10⁻⁶ M). La quantification d'AMP cyclique est déterminée par dosage radio-immunologique. Les données représentent la moyenne ± ESM (n = 3).
**Figure 6** **: Effet du composé I sur l'activité basale de la MAP kinase dans les cellules MCF7.**
   Les cellules, privées en sérum pendant 48 heures sont traitées pendant 1 heure par le composé I (10 et 50 µM). L'activité de la MAP kinase est déterminée dans les lysats cellulaires et est exprimée en pmole de Pi incorporé / minute / 4 µg de protéine.(n = 3 pour les échantillons témoins et n = 2 pour les cellules traitées par le composé I).
**Figure 7** **: Effet du composé I comparé au PD98059 sur l'activité de la MAP kinase dans les cellules MCF7.**
   Les cellules, privées en sérum pendant 48 heures sont traitées pendant 1 heure soit par le composé 1 (10 et 50 µM) soit par le PD98059 (10 et 50 µM). Les cellules sont ensuite stimulées par l'addition d'EGF (12,5 ng/ml) pendant 5 minutes. L'activité de la MAP kinase est déterminée dans les lysats cellulaires et est exprimée en pmole de Pi incorporé / minute / 4 µg de protéine.(n = 4).

## Revendications

1. Utilisation d'un dérivé de cystéine de formule générale (A) : 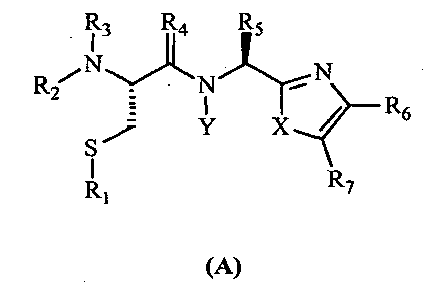 correspondant aux sous-formules **(A1)** ou **(A2)** : 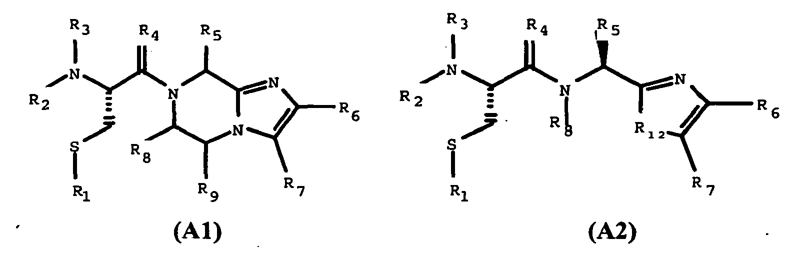 dans lesquelles :
X représente R₁₂ et Y représente R₈, ou X et Y complètent un cycle à 6 chaînons, l'ensemble X-Y représentant le radical -CH(R₈)-CH(R₉)- ;
R₁ représente H, un radical alkyle comptant de 1 à 6 atomes de carbone ou alkylthiocomptant de 1 à 6 atomes de carbone ;
R₂ et R₃ représentent indépendamment H ou un radical alkyle comptant de 1 à 6 atomes de carbone ;
R₄ représente H₂ ou O ;
R₅ représente H, ou l'un des radicaux alkyle comptant de 1 à 6 atomes de carbone, alkényle comptant jusqu'à 6 atomes de carbone, alkynyle comptant jusqu'à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé d'un radical alkyle comptant de 1 à 6 atomes de carbone, -OR₁₀, -S(O)ₘR₁₀ (m représentant 0, 1, ou 2), -N(R₁₀)(R₁₁), -N-C(O)-R₁₀, -NH-(SO₂)-R₁₀, -CO₂-R₁₀, C(O)-N(R₁₀)(R₁₁), et -(SO₂)-N(R₁₀)(R₁₁) ;
R₆ et R₇ représentent indépendamment H, un radical -C(O)-NH-CHR₁₃-CO₂R₁₄, ou l'un des radicaux alkyle comptant de 1 à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé des radicaux OH, alkyle ou alkoxy comptant de 1 à 6 atomes de carbone, N(R₁₀)(R₁₁), COOH, CON(R₁₀)(R₁₁), et halo,
ou R₆ et R₇ forment ensemble un radical aryle ou un hétérocycle ;
R₈ et R₉ représentent indépendamment, H, ou l'un des radicaux alkyle comptant de 1 à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé des radicaux OH, alkyle ou alkoxy comptant de 1 à 6 atomes de carbone, N(R₁₀)(R₁₁), COOH, CON(R₁₀)(R₁₁) et halo,
ou R₈ et R₉ forment ensemble un radical aryle ou un hétérocycle ;
R₁₀ et R₁₁, représentent indépendamment H, un radical aryle ou hétérocycle, ou un radical alkyle comptant de 1 à 6 atomes de carbone, arylalkyle dont le radical alkyle compte de 1 à 6 atomes de carbone ou hétérocycle alkyle dont le radical alkyle compte de 1 à 6 atomes de carbone ;
R₁₂ représente NR₉, S, ou O ;
R₁₃ représente un radical alkyle inférieur éventuellement substitué par un radical choisi parmi les radicaux alkyle comptant de 1 à 6 atomes de carbone, -OR₁₀, -S(O)ₘR₁₀ (m représentant 0, 1, ou 2) et -N(R₁₀)(R₁₁);
R₁₄ représente H ou un radical alkyle comptant de 1 à 6 atomes de carbone ;
étant entendu que les composés de formule générale (A) peuvent également se présenter sous forme de dimères, lorsque deux radicaux R₁ représentant chacun un atome d'hydrogène sont éliminés par oxydation ;
ou d'un sel pharmaceutiquement acceptable d'un composé de formule générale **(A) ;**
pour préparer un médicament destiné à traiter des pathologies qui résultent de la formation de la protéine G hétérotrimérique, **caractérisée en ce que** la pathologie à traiter est choisie parmi des pathologies liées aux fonctions biologiques ou désordres suivants : odorat, goût, perception de la lumière, neurotransmission, neurodégénérescence, fonctionnement des glandes endocrines et exocrines, régulation autocrine et paracrine, tension artérielle, embryogénèse, infection virale, fonctions immunologiques, diabète et obésité, mais également parmi les pathologies suivantes : le choléra, le Syndrome Immuno-Déficitaire Acquis (SIDA), la diarrhée du voyageur et la puberté précoce familiale masculine.

2. Utilisation selon la revendication 1, **caractérisée en ce que** le composé utilisé est choisi parmi l'un des composés suivants :
- la 7-(2-amino-1-oxo-3-thiopropyl)-8-(cyclohexylméthyl)-2-(2-méthylphényl)-5,6,7,8-tétrahydroimidazo[1,2a]pyrazine ;
- la 7-(2-amino-1-oxo-3-thiopropyl)-8-butyl-2-(2-méthoxyphényl)-5,6,7,8-tétrahydroimidazo[1,2a]pyrazine ;
- la 7-(2-amino-1-oxo-3-thiopropyl)-8-(1-méthylpropyl)-2-(2-méthoxyphényl)-5,6,7,8-tétrahydroimidazo[1,2a]pyrazine ;
- le disulfure de bis-1,1'-[7-(2-amino-1-oxo-3-thiopropyl)-2-(méthoxyphényl)-8-(1-méthylpropyl)-5,6,7,8-tétrahydroimidazo[1,2a]pyrazine] ;
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine ;
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) ;
- la 7-(2-amino-1-oxo-3-thiopropyl)-8-(cyclohexylméthyl)-2-phényl-5,6,7,8-tétrahydroimidazo[1,2a]pyrazine ;
- la 7-(2-amino-1-oxo-3-thiopropyl)-2-(2-méthoxyphényl)-8-(phénylméthoxy)méthyl-5,6,7,8-tétrahydroimidazo[1,2a]pyrazine ;
- la 7-(2-amino-1-oxo-3-thiopropyl)-2-(2-méthoxyphényl)-8-(1-phénylméthoxy)éthyl-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydro-imidazo[1,2a]pyrazine, ou sa forme dimérique ;
- et la 7-(2-amino-1-oxo-3-thiopropyl)-2-(2-méthoxyphényl)-8-(phénylsulfonyléthyl)-5,6,7,8-tétrahydro-imidazo[1,2a]pyrazine ;
- ou un sel pharmaceutiquement acceptable d'un de ces derniers.

3. Utilisation selon la revendication 2, **caractérisée en ce que** le composé utilisé est choisi parmi l'un des composés suivants :
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine **(I)** ;
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) **(II) ;**
- la 7-(2-amino-1-oxo-3-thiopropyl)-8-(cyclohexylméthyl)-2-(2-méthylphényl)-5,6,7,8-tétrahydroimidazo[1,2a]pyrazine **(III) ;**
- la 7-(2-amino-1-oxo-3-thiopropyl)-8-butyl-2-(2-méthoxyphényl)-5,6,7,8-tétrahydroimidazo[1,2a]pyrazine **(V) ;**
- le disulfure de bis-1,1'-[7-(2-amino-1-oxo-3-thiopropyl)-2-(méthoxyphényl)-8-(1-méthylpropyl)-5,6,7,8-tétrahydroimidazo[1,2a]pyrazine] **(VI) ;**
- la 7-(2-amino-1-oxo-3-thiopropyl)-8-(cyclohexylméthyl)-2-phényl-5,6,7,8-tétrahydroimidazo[1,2a]pyrazine ;
- la 7-(2-amino-1-oxo-3-thiopropyl)-8-(1-méthylpropyl)-2-(2-méthoxyphényl)-5,6,7,8-tétrahydroimidazo[1,2a]pyrazine ;
- la 1-[2(R)-amino-3-mercaptopropyl]-2(S)-n-butyl-4-(1-naphtoyl)pipérazine ;
- ou un sel pharmaceutiquement acceptable d'un de ces derniers.

4. Utilisation selon la revendication 3, **caractérisée en ce que** le composé utilisé est choisi parmi l'un des composés suivants :
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine **(I) ;**
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) **(II) ;**
ou un sel pharmaceutiquement acceptable d'un de ces derniers.

5. Utilisation du 7-(2-amino-1-oxo-3-thiopropyl)-8-(cyclohexylmethyl)-2-phenyl-5,6,7,8-tetrahydroimidazo[1,2a]pyrazine ou d'un sel pharmaceutiquement acceptable de ce composé pour la préparation d'un médicament **caractérisé en ce que** la pathologie à traiter est choisie parmi les fonctions biologiques ou désordres suivants : odorat, goût, perception de la lumière, neurotransmission, neurodégénérescence, fonctionnement des glandes endocrines et exocrines, régulation autocrine et panacrine, tension artérielle, embryogénèse, infection virale, fonctions immunologiques, diabète, obésité, choléra, le Syndrome Immuno-Déficitaire Acquis (SIDA), la diarrhée du voyageur et la puberté précoce familiale masculine.

6. Utilisation selon l'une des revendications 1 à 4, **caractérisée en ce que** la pathologie à traiter est choisie parmi des pathologies liées aux fonctions biologiques ou désordres suivants : odorat, goût, perception de la lumière, neurotransmission, neurodégénérescence, fonctionnement des glandes endocrines et exocrines, régulation autocrine et paracrine, tension artérielle, embryogénèse, infection virale, fonctions immunologiques, diabète et obésité.

7. Utilisation selon l'une des revendications 1 à 4, **caractérisée en ce que** la pathologie à traiter est choisie parmi les pathologies suivantes : le choléra, le Syndrome Immuno-Déficitaire Acquis (SIDA), la diarrhée du voyageur et la puberté précoce familiale masculine.

## Claims

1. Use of a cysteine derivative, of general formula (A): 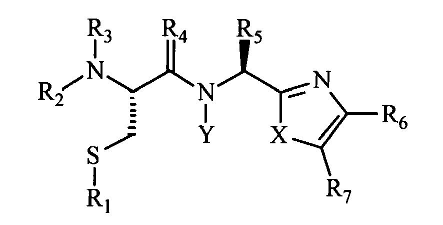 corresponding to sub-formulae **(A1)** or **(A2):** 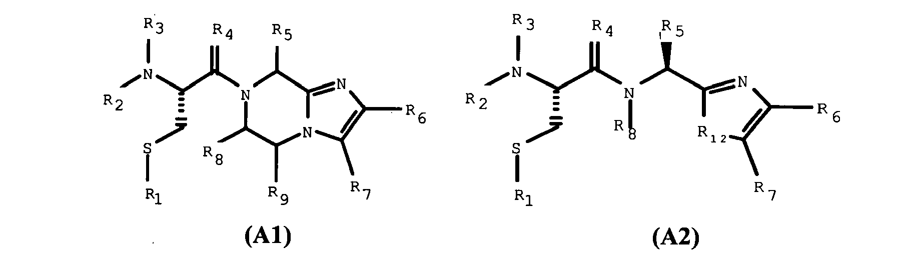 in which:
X represents R₁₂ and Y represents R₈, or X and Y complete a ring with 6 members, the X-Y set representing the -CH(R₈)-CH(R₉)- radical;
R₁ represents H, an alkyl radical comprising 1 to 6 carbon atoms or alkylthio radical comprising 1 to 6 carbon atoms;
R₂ and R₃ represent independently H or an alkyl radical comprising 1 to 6 carbon atoms; R₄ represents H₂ or O;
R₅ represents H, or one of the alkyl comprising 1 to 6 carbon atoms, alkenyl comprising up to 6 carbon atoms, alkynyl comprising up to 6 carbon atoms, aryl, arylalkyl the alkyl of which comprising 1 to 6 carbon atoms, heterocycle or alkyl heterocycle the alkyl of which comprising 1 to 6 carbon atoms, radicals, these radicals being optionally substituted by radicals chosen from the group comprising an alkyl radical comprising 1 to 6 carbon atoms, -O-R₁₀, -S(O)ₘR₁₀ (m representing 0, 1, or 2), -N(R₁₀)(R₁₁), -N-C(O)-R₁₀, -NH-(SO₂)-R₁₀, -CO₂-R₁₀, C(O)-N(R₁₀)(R₁₁), and -(SO₂)-N(R₁₀)(R₁₁);
R₆ and R₇ represent independently H, a -C(O)-NH-CHR₁₃-CO₂R₁₄ radical, or one of the alkyl comprising 1 to 6 carbon atoms, aryl, arylalkyl the alkyl of which comprising 1 to 6 carbon atoms, heterocycle or alkyl heterocycle the alkyl of which comprising 1 to 6 carbon atoms, radicals, these radicals being optionally substituted by radicals chosen from the group comprising the OH, alkyl or alkoxy comprising 1 to 6 carbon atoms, N(R₁₀)(R₁₁), COOH, CON(R₁₀)(R₁₁), and halo radicals,
or R₆ and R₇ form together an aryl radical or a heterocycle;
R₈ and R₉ represent independently, H, or one of the alkyl comprising 1 to 6 carbon atoms, aryl, arylalkyl the alkyl of which comprising 1 to 6 carbon atoms, heterocycle or alkyl heterocycle the alkyl of which comprising 1 to 6 carbon atoms, radicals, these radicals being optionally substituted by radicals chosen from the group comprising the OH, alkyl or alkoxy comprising 1 to 6 carbon atoms, N(R₁₀)(R₁₁), COOH, CON(R₁₀)(R₁₁) and halo radicals, or R₈ and R₉ together form an aryl radical or a heterocycle;
R₁₀ and R₁₁ represent independently H, an aryl radical or a heterocycle, or an alkyl comprising 1 to 6 carbon atoms, arylalkyl the alkyl of which comprising 1 to 6 carbon atoms or alkyl heterocycle radical the alkyl of which comprising 1 to 6 carbon atoms;
R₁₂ represents NR₉, S, or O;
R₁₃ represents an alkyl radical comprising 1 to 6 carbon atoms optionally substituted by a radical chosen from the alkyl comprising 1 to 6 carbon atoms, -OR₁₀, -S(O)ₘR₁₀ (m representing 0, 1, or 2) and -N(R₁₀)(R₁₁) radicals;
R₁₄ represents H or a alkyl radical comprising 1 to 6 carbon atoms;
it being understood that the compounds of general formula (A) can also be presented in the form of dimers, when two R₁ radicals each representing a hydrogen atom are eliminated by oxidation;
or of a pharmaceutically acceptable salt of a compound of general formula (A);
for preparing a medicament intended to treat pathologies which result from the formation of the heterotrimeric G protein **characterized in that** the pathology to be treated is chosen from the pathologies linked to the following biological functions or disorders: smell, taste, perception of light, neurotransmission, neurodegeneration, endocrine and exocrine gland functions, autocrine and paracrine regulation, arterial tension, embryogenesis, viral infection, immunological functions, diabetes and obesity and also from the following pathologies: cholera, Acquired Immune Deficiency Syndrome (AIDS), travel diarrhea and familial masculine precocious puberty.

2. Use according to claim 1, **characterized in that** the compound used is chosen from one of the following compounds:
- 7-(2-amino-1-oxo-3-thiopropyl)-8-(cyclohexylmethyl)-2-(2-methylphenyl)-5,6,7,8-tetrahydroimidazo[1,2a]pyrazine;
- 7-(2-amino-1-oxo-3-thiopropyl)-8-butyl-2-(2-methoxyphenyl)-5,6,7,8-tetrahydroimidazo[1,2a]pyrazine;
- 7-(2-amino-1-oxo-3-thiopropyl)-8-(1-methylpropyl)-2-(2-methoxyphenyl)-5,6,7,8-tetrahydroimidazo[1,2a]pyrazine;
- bis-1,1'-[7-(2-amino-1-oxo-3-thiopropyl)-2-(methoxyphenyl)-8-(1-methylpropyl)-5,6,7,8-tetrahydroimidazo[1,2a]pyrazine] disulphide;
- bis-1,1'-[7-(2-amino-1-oxo-3-thiopropyl)-8-(cyclohexylmethyl)-2-(2-methoxyphenyl)-5,6,7,8-tetrahydroimidazo[1,2a]pyrazine disulphide;
- bis-1,1'-7-(2-amino-1-oxo-3-thiopropyl-(2-(1-naphthyl)-8-(2-methylpropyl)-5,6,7,8-tetrahydroimidazo[1,2a]pyrazin-7-yl) disulphide;
- 7-(2-amino-1-oxo-3-thiopropyl)-8-(cyclohexylmethyl)-2-phenyl-5,6,7,8-tetrahydroimidazo[1,2a]pyrazine;
- 7-(2-amino-1-oxo-3-thiopropyl)-2-(2-methoxyphenyl)-8-(phenylmethoxy)methyl-5,6,7,8-tetrahydroimidazo[1,2a]pyrazine;
- 7-(2-amino-1-oxo-3-thiopropyl)-2-(2-methoxyphenyl)-8-(1-phenylmethoxy)ethyl-5,6,7,8-tetrahydroimidazo[1,2a]pyrazine;
- 7-(2-amino-1-oxo-3-thiopropyl)-2-(2-methoxyphenyl)-8-(phenoxyethyl)-5,6,7,8-tetrahydroimidazo[1,2a]pyrazine;
- 7-(2-amino-1-oxo-3-thiopropyl)-2-(2-methyoxyphenyl)-8-(phenoxyethyl)-5,6,7,8-tetrahydro-imidazo[1,2a]pyrazine, or its dimeric form;
- and 7-(2-amino-1-oxo-3-thiopropyl)-2-(2-methoxyphenyl)-8-(phenylsulphonylethyl)-5,6,7,8-tetrahydro-imidazo[1,2a]pyrazine;
- or a pharmaceutically acceptable salt of one of the latter.

3. Use according to claim 2, **characterized in that** the compound used is chosen from one of the following compounds:
- bis-1,1'-[7-(2-amino-1-oxo-3-thiopropyl)-8-(cyclohexylmethyl)-2-(2-methoxyphenyl)-5,6,7,8-tetrahydroimidazo[1,2a]pyrazine disulphide **(I);**
- bis-1,1'-7-(2-amino-1-oxo-3-thiopropyl-(2-(1-naphtyl)-8-(2-methylpropyl)-5,6,7,8-tetrahydroimidazo[1,2a]pyrazin-7-yl) disulphide **(II);**
- 7-(2-amino-1-oxo-3-thiopropyl)-8-(cyclohexylmethyl)-2-(2-methylphenyl)-5,6,7,8-tetrahydroimidazo[1,2a]pyrazine **(III);**
- 7-(2-amino-1-oxo-3-thiopropyl)-8-butyl-2-(2-methoxyphenyl)-5,6,7,8-tetrahydroimidazo[1,2a]pyrazine **(V);**
- bis-1,1'-[7-(2-amino-1-oxo-3-thiopropyl)-2-(methoxyphenyl)-8-(1-methylpropyl)-5,6,7,8-tetrahydroimidazo[1,2a]pyrazine] disulphide **(VI);**
- 7-(2-amino-1-oxo-3-thiopropyl)-8-(cyclohexylmethyl)-2-phenyl-5,6,7,8-tetrahydroimidazo[1,2a]pyrazine;
- 7-(2-amino-1-oxo-3-thiopropyl)-8-(1-methylpropyl)-2-(2-methoxyphenyl)-5,6,7,8-tetrahydroimidazo[1,2a]pyrazine;
- 1-[2(R)-amino-3-mercaptopropyl]-2(S)-n-butyl-4-(1-naphthoyl)piperazine;
- or a pharmaceutically acceptable salt of one of the latter.

4. Use according to claim 3, caracterized in that the compound used is chosen from one of the following compounds:
- bis-1,1'-[7-(2-amino-1-oxo-3-thiopropyl)-8-(cyclohexylmethyl)-2-(2-methoxyphenyl)-5,6,7,8-tetrahydroimidazo[1,2a]pyrazine disulphide **(I);**
- bis-1,1'-7-(2-amino-1-oxo-3-thiopropyl-(2-(1-naphtyl)-8-(2-methylpropyl)-5,6,7,8-tetrahydroimidazo[1,2a]pyrazin-7-yl) disulphide **(II);**
- or a pharmaceutically acceptable salt of one of the latter.

5. Use of - 7-(2-amino-1-oxo-3-thiopropyl)-8-(cyclohexylmethyl)-2-phenyl-5,6,7,8-tetrahydroimidazo[1,2a]pyrazine or of a pharmaceutically acceptable salt of one of this compound for preparing a medicament **characterized in that** pathology to treat is chosen from the following biological functions or disorders: smell, taste, perception of light, neurotransmission, neurodegeneration, endocrine and exocrine gland functions, autocrine and paracrine regulation, arterial tension, embryogenesis, viral infection, immunological functions, diabetes, obesity, cholera, Acquired Immune Deficiency Syndrome (AIDS), travel diarrhea and familial masculine precocious puberty;

6. Use according to one of claims 1 to 4, **characterized in that** the pathology to be treated is chosen from the pathologies linked to the following biological functions or disorders: smell, taste, perception of light, neurotransmission, neurodegeneration, endocrine and exocrine gland functions, autocrine and paracrine regulation, arterial tension, embryogenesis, viral infection, immunological functions, diabetes and obesity.

7. Use according to one of claims 1 to 4, **characterized in that** the pathology to be treated is chosen from the following pathologies: cholera, Acquired Immune Deficiency Syndrome (AIDS), travel diarrhea and familial masculine precocious puberty.

## Patentansprüche

1. Verwendung eines Cystein-Derivats der allgemeinen Formel (A): 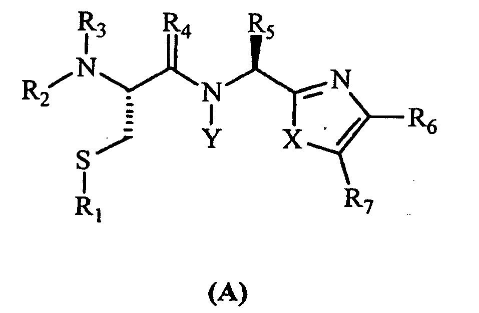 die den Unterformeln (A1) oder (A2) entspricht: 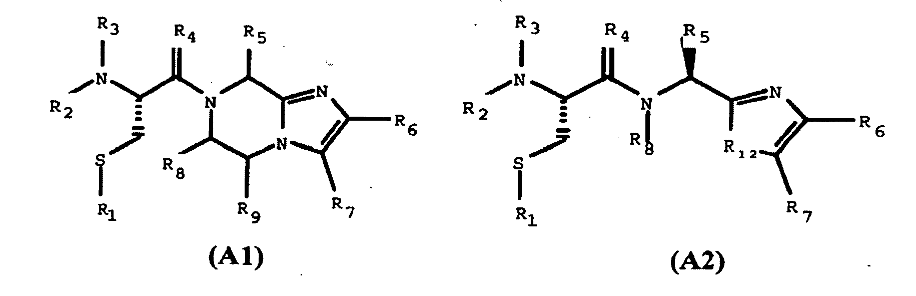 in denen:
X R₁₂ darstellt und Y R₈ darstellt oder X und Y einen Ring mit 6 Kettengliedern vervollständigen, die Kombination X-Y den Rest -CH(R₈)-CH(R₉)- darstellt;
R₁ H, einen Alkyl-Rest mit 1 bis 6 Kohlenstoffatomen oder einen Alkylthio-Rest mit 1 bis 6 Kohlenstoffatomen darstellt;
R₂ und R₃ unabhängig H oder einen Alkyl-Rest mit 1 bis 6 Kohlenstoffatomen darstellen;
R₄ H₂ oder O darstellt;
R₅ H oder einen der Reste Alkyl mit 1 bis 6 Kohlenstoffatomen, Alkenyl mit bis zu 6 Kohlenstoffatomen, Alkinyl mit bis zu 6 Kohlenstoffatomen, Aryl, Arylalkyl, dessen Alkyl-Rest 1 bis 6 Kohlenstoffatome hat, Heterocyclyl oder Heterocycloalkyl, dessen Alkyl-Rest 1 bis 6 Kohlenstoffatome hat, darstellt, wobei diese Reste gegebenenfalls mit Resten substituiert sein können, die ausgewählt sind aus der Gruppe, die aus einem Alkyl-Rest mit 1 bis 6 Kohlenstoffatomen, -O-R₁₀, -S(O)ₘR₁₀ (m stellt 0, 1 oder 2 dar), -N(R₁₀)(R₁₁), -N-C (O) -R₁₀, -NH-(SO₂)-R₁₀, -CO₂-R₁₀, C (O) - N(R₁₀)(R₁₁) und -(SO₂)-N(R₁₀)(R₁₁) besteht;
R₆ und R₇ unabhängig H, einen Rest -C(O)-NH-CHR₁₃-CO₂R₁₄ oder einen der Reste Alkyl mit 1 bis 6 Kohlenstoffatomen, Aryl, Arylalkyl, dessen Alkyl-Rest 1 bis 6 Kohlenstoffatome hat, Heterocyclyl oder Heterocycloalkyl, dessen Alkyl-Rest 1 bis 6 Kohlenstoffatome hat, darstellen, wobei diese Reste gegebenenfalls durch Reste substituiert sein können, die ausgewählt sind aus der Gruppe, die aus den Resten OH, Alkyl oder Alkoxy mit 1 bis 6 Kohlenstoffatomen, N(R₁₀)(R₁₁), COOH, CON(R₁₀)(R₁₁) und Halogen besteht;
oder R₆ und R₇ zusammen einen Aryl-Rest oder einen Heterocyclyl-Rest bilden;
R₈ und R₉ unabhängig voneinander H oder einen der Reste Alkyl mit 1 bis 6 Kohlenstoffatomen, Aryl, Arylalkyl, dessen Alkyl-Rest 1 bis 6 Kohlenstoffatome hat, Heterocyclyl oder Heterocycloalkyl, dessen Alkyl-Rest 1 bis 6 Kohlenstoffatome hat, darstellen, wobei diese Reste gegebenenfalls mit Resten substituiert sein können, die unabhängig ausgewählt sind aus der Gruppe, die aus den Resten OH, Alkyl oder Alkoxy mit 1 bis 6 Kohlenstoffatomen, N(R₁₀)(R₁₁), COOH, CON(R₁₀)(R₁₁) und Halogen besteht,
oder R₈ und R₉ zusammen einen Aryl-Rest oder einen Heterocyclyl-Rest bilden;
R₁₀ und R₁₁ unabhängig H, einen Aryl- oder Heterocyclyl-Rest oder einen Alkyl-Rest mit 1 bis 6 Kohlenstoffatomen, einen Arylalkyl-Rest, dessen Alkyl-Rest 1 bis 6 Kohlenstoffatome hat, oder einen Heterocycloalkyl-Rest, dessen Alkyl-Rest 1 bis 6 Kohlenstoffatome hat, darstellen;
R₁₂ NR₉, S oder O darstellt;
R₁₃ einen Niederalkyl-Rest darstellt, der gegebenenfalls mit einem Rest substituiert ist, der ausgewählt ist aus den Resten: Alkyl mit 1 bis 6 Kohlenstoffatomen, -OR₁₀,
-S(O)ₘR₁₀ (m stellt 0, 1 oder 2 dar) und -N(R₁₀)(R₁₁) ;
R₁₄ H oder einen Alkyl-Rest mit 1 bis 6 Kohlenstoffatomen darstellt;
mit der Maßgabe, dass die Verbindungen der allgemeinen Formel (A) auch in Form von Dimeren vorliegen können, wenn zwei Reste R₁, die jeweils ein Wasserstoffatom darstellen, durch Oxidation eliminiert sind;
oder eines pharmazeutisch verträglichen Salzes einer Verbindung der allgemeinen Formel (A);
zur Herstellung eines Medikaments, das zur Behandlung von Pathologien bestimmt ist, die aus der Bildung des heterotrimeren Proteins G resultieren, **dadurch gekennzeichnet, dass** die zu behandelnde Pathologie aus Pathologien, die mit den folgenden biologischen Funktionen oder Störungen verbunden sind: Geruchsinn, Geschmack, Lichtwahrnehmung, Neurotransmission, Neurodegeneration, Funktion der endokrinen und exokrinen Drüsen, autokrine und parakrine Regulation, arterieller Druck, Embryogenese, virale Infektion, Immunfunktionen, Diabetes und Adipositas, aber auch aus den folgenden Pathologien: Cholera, erworbenes Immundefizienz-Syndrom (AIDS), Reisediarrhöe und familiäre männliche Pubertas praecox, ausgewählt sind.

2. Verwendung gemäß Anspruch 1, **dadurch gekennzeichnet, dass** die verwendete Verbindung aus den folgenden Verbindungen:
- 7-(2-Amino-1-oxo-3-thiopropyl)-8-(cyclohexylmethyl)-2-(2-methylphenyl)-5,6,7,8-tetrahydroimidazo[1,2a]-pyrazin;
- 7-(2-Amino-1-oxo-3-thiopropyl)-8-butyl-2-(2-methoxyphenyl)-5,6,7,8-tetrahydroimidazo[1,2a]pyrazin;
- 7-(2-Amino-1-oxo-3-thiopropyl)-8-(1-methylpropyl)-2-(2-methoxyphenyl)-5,6,7,8-tetrahydroimidazo[1,2a]-pyrazin;
- Bis-1,1'-[7-(2-amino-1-oxo-3-thiopropyl)-2-(methoxyphenyl)-8-(1-methylpropyl)-5,6,7,8-tetrahydroimidazo-[1,2a]pyrazin]-disulfid;
- Bis-1,1'-[7-(2-amino-1-oxo-3-thiopropyl)-8-(cyclohexylmethyl)-2-(2-methoxyphenyl)-5,6,7,8-tetrahydro-imidazo[1,2a]pyrazin]-disulfid;
- Bis-1,1'-7-(2-amino-1-oxo-3-thiopropyl-(2-(1-naphthyl)-8-(2-methylpropyl)-5,6,7,8-tetrahydroimidazo-[1,2a]pyrazin-7-yl)-disulfid;
- 7-(2-Amino-1-oxo-3-thiopropyl)-8-(cyclohexylmethyl)-2-phenyl-5,6,7,8-tetrahydroimidazo[1,2a]pyrazin;
- 7-(2-Amino-1-oxo-3-thiopropyl)-2-(2-methoxyphenyl)-8-(phenylmethoxy)methyl-5,6,7,8-tetrahydroimidazo-[1,2a]pyrazin;
- 7-(2-Amino-1-oxo-3-thiopropyl)-2-(2-methoxyphenyl)-8-(1-phenylmethoxy)ethyl-5,6,7,8-tetrahydroimidazo-[1,2a]pyrazin;
- 7-(2-Amino-1-oxo-3-thiopropyl)-2-(2-methoxyphenyl)-8-(phenoxyethyl)-5,6,7,8-tetrahydroimidazo[1,2a]-pyrazin;
- 7-(2-Amino-1-oxo-3-thiophenyl)-2-(2-methoxyphenyl)-8-(phenoxyethyl)-5,6,7,8-tetrahydroimidazo[1,2a] oder seiner dimeren Form; und
- 7-(2-Amino-1-oxo-3-thiopropyl)-2-(2-methoxyphenyl)-8-(phenylsulfonylethyl)-5,6,7,8-tetrahydroimidazo-[1,2a]pyrazin;
oder einem pharmazeutisch verträglichen Salz einer dieser letztgenannten ausgewählt ist.

3. Verwendung gemäß Anspruch 2, **dadurch gekennzeichnet, dass** die verwendete Verbindung aus den folgenden Verbindungen
- Bis-1,1'-[7-(2-amino-1-oxo-3-thiopropyl)-8-(cyclohexylmethyl)-2-(2-methoxyphenyl)-5,6,7,8-tetra-hydroimidazo[1,2a]pyrazin-disulfid **(I);**
- Bis-1,1'-7-(2-amino-1-oxo-3-thiopropyl-(2-(1-naphthyl)-8-(2-methylpropyl)-5,6,7,8-tetrahydro-imidazo[1,2a]pyrazin-7-yl)-disulfid **(II) ;**
- 7-(2-Amino-1-oxo-3-thiopropyl)-8-(cyclohexylmethyl)-2-(2-methylphenyl)-5,6,7,8-tetrahydroimidazo-[1,2a]pyrazin **(III) ;**
- 7-(2-Amino-1-oxo-3-thiopropyl)-8-butyl-2-(2-methoxyphenyl)-5,6,7,8-tetrahydroimidazo[1,2a]pyrazin **(V);**
- Bis-1,1'-[7-(2-amino-1-oxo-3-thiopropyl)-2-(methoxyphenyl)-8-(1-methylpropyl)-5,6,7,8-tetrahydroimidazo-[1,2a]pyrazin]-disulfid **(VI);**
- 7-(2-Amino-1-oxo-3-thiopropyl)-8-(cyclohexylmethyl)-2-phenyl-5,6,7,8-tetrahydroimidazo[1,2a]pyrazin;
- 7-(2-Amino-1-oxo-3-thiopropyl)-8-(1-methylpropyl)-2-(2-methoxyphenyl)-5,6,7,8-tetrahydroimidazo-[1,2a]pyrazin;
- 1-[2(R)-Amino-3-mercaptopropyl]-2(S)-n-butyl-4-(1-naphthoyl)piperazin;
oder einem pharmazeutisch verträglichen Salz einer dieser letztgenannten ausgewählt ist.

4. Verwendung gemäß Anspruch 3, **dadurch gekennzeichnet, dass** die verwendete Verbindung aus den folgenden Verbindungen:
- Bis-1,1'-[7-(2-amino-1-oxo-3-thiopropyl)-8-(cyclohexylmethyl)-2-(2-methoxyphenyl)-5,6,7,8-tetrahydro-imidazo[1,2a]pyrazin-disulfid **(I);**
- Bis-1,1'-7-(2-amino-1-oxo-3-thiopropyl-(2-(1-naphthyl)-8-(2-methylpropyl)-5,6,7,8-tetrahydroimidazo-[1,2a]pyrazin-7-yl)-disulfid **(II) ;**
oder einem pharmazeutisch verträglichen Salz einer dieser letztgenannten ausgewählt ist.

5. Verwendung von 7-(2-Amino-1-oxo-3-thiopropyl)-8-(cyclohexylmethyl)-2-phenyl-5,6,7,8-tetrahydroimidazo[1,2a]pyrazin oder eines pharmazeutisch verträglichen Salzes dieser Verbindung zur Herstellung eines Medikaments, **dadurch gekennzeichnet, dass** die zu behandelnde Pathologie aus den folgenden biologischen Funktionen oder Störungen ausgewählt ist: Geruchsinn, Geschmack, Lichtwahrnehmung, Neurotransmission, Neurodegeneration, Funktion der endokrinen und exokrinen Drüsen, autokrine und parakrine Regulation, arterieller Druck, Embryogenese, virale Infektion, Immunfunktionen, Diabetes, Adipositas, Cholera, erworbenes Immundefizienz-Syndrom (AIDS), Reisediarrhöe und familiäre männliche Pubertas praecox.

6. Verwendung gemäß einem der Ansprüche 1 bis 4, **dadurch gekennzeichnet, dass** die zu behandelnde Pathologie unter Pathologien ausgewählt ist, die mit den folgenden biologischen Funktionen oder Störungen verbunden sind: Geruchsinn, Geschmack, Lichtwahrnehmung, Neurotransmission, Neurodegeneration, Funktion der endokrinen und exokrinen Drüsen, autokrine und parakrine Regulation, arterieller Druck, Embryogenese, virale Infektion, Immunfunktionen, Diabetes und Adipositas.

7. Verwendung gemäß einem der Ansprüche 1 bis 4, **dadurch gekennzeichnet, dass** die zu behandelnde Pathologie unter den folgenden Pathologien ausgewählt ist: Cholera, erworbenes Immundefizienz-Syndrom (AIDS), Reisediarrhöe und familiäre männliche Pubertas praecox.
